# EUROPEAN PATENT APPLICATION

(11) **EP 3 901 171 A1**
(43) Date of publication of application: **27.10.2021**
(21) Application number: 19889992.4
(22) Date of filing: 02.12.2019
(51) Int. Cl.: C07K 16/28, C07K 19/00, C12N 15/13, C12N 15/63, C12N 5/078, C12N 5/10, A61K 35/15, A61K 35/17, A61P 35/00, A61P 37/06

(54) **ANTI-HUMAN TIM-3 MONOCLONAL ANTIBODY AND APPLICATION THEREOF**

(30) Priority: 30.11.2018 CN 201811459248
(71) Applicant: Shanghai Pharmaexplorer Co., Ltd., Shanghai 201210 (CN); Pharmaexplorer Limited, Road Town, Tortola (VG)
(72) Inventor: SONG, Ningning, Shanghai 201210 (CN); DUAN, Qing, Shanghai 201210 (CN); LIU, Lile, Shanghai 201210 (CN); YANG, Tatchi Teddy, Shanghai 201210 (CN); XU, Lina, Shanghai 201210 (CN); LIU, Hu, Shanghai 201210 (CN); WEI, Peipei, Shanghai 201210 (CN); WANG, Qian, Shanghai 201210 (CN); WANG, Yuandong, Shanghai 201210 (CN); SHAO, Xiaohui, Shanghai 201210 (CN); HU, Shaoping, Shanghai 201210 (CN); ZHANG, Yu, Shanghai 201210 (CN); WU, Jian, Shanghai 201210 (CN); WANG, Meiling, Shanghai 201210 (CN); WANG, Dongxu, Shanghai 201210 (CN); DAI, Chaohui, Shanghai 201210 (CN); WANG, Mengying, Shanghai 201210 (CN)
(74) Representative: Mewburn Ellis LLP
(86) International application number: PCT/CN2019/122471
(87) International publication number: WO 2020/108660

(57) **Abstract**

Provided are a high-affinity full human monoclonal antibody highly specifically targeting to TIM-3 and a preparation method therefor. The monoclonal antibody has a remarkable antitumor activity, and can be used for preparing a related diagnostic reagent or a pharmaceutical composition preventing or treating diseases related to TIM-3 dysfunction.

## Description

### Technical field

The invention relates to the biomedical field, in particular to a TIM-3 antibody as well as preparation method and application thereof.

### Background

T cell immunoglobulin domain and mucin domain 3 (TIM-3, HAVCR2) is a type I membrane protein having 301 amino acids, which is one of the major known immune checkpoints. TIM-3 is mainly expressed in activated CD4⁺ and CD8⁺ T lymphocytes, natural regulatory T cells, NK cells and congenital cells (macrophages, monocytes and dendritic cells). It has been reported that the ligands of TIM-3 include phosphatidylserine (PtdSer), galectin-9 (Gal-9), high mobility group protein 1 (HMGB1) and carcino-embryonic antigen cell adhesion molecule 1 (CECAM1). TIM-3 plays a role in regulating various aspects of immune response by binding with its ligand. The interaction between TIM-3 and CECAM1 inhibits T cell activation and promotes T cell tolerance and depletion. Regulatory T cells and many tumor cells express Gal-9 protein and combine with TIM-3 to inhibit the activation of effector T cells. The combination of HMGB1 and TIM-3 acts on antigen-presenting cells and congenital cells to reduce inflammatory reactions mediated by injury-related molecular patterns. TIM-3 combined with phosphatidylserine promotes the clearance of apoptotic cells.

Studies have shown that the abnormal expression of TIM-3 is closely related to many diseases. Some studies have found that the expression of TIM-3 in T cells of patients infected with HIV and HCV and some tumor patients is increased, which mediates the apoptosis of T effector cells and transmits negative regulatory signals, thus leading to the disorder of immune system and paralysis of immune function. In addition, tumor-infiltrating lymphocytes in various mouse tumor models co-express TIM-3 and PD-1, and exhibit functional depletion, loss of proliferation, and ability of secretion of some cytokines (IL-2, TNF-α and IFN-γ). Blocking PD-1 and TIM-3 signaling pathway at the same time can inhibit tumor growth more effectively than blocking PD-1 or TIM-3 signaling pathway alone. In many human tumor-infiltrating T cells, compared with TIM-3⁻/PD-1⁺ and TIM -3⁻/PD-1⁻CD8⁺ T cells, TIM-3⁺/PD-1⁺ double positive CD8⁺ T cells are more dysfunctional and the ability to secrete cytokines is weaker.

At present, only two TIM-3 antibodies at home and abroad are in the clinical research stage, other antibody drugs are still in the discovery and research stage, and no TIM-3 antibody has yet entered clinical application. It is urgent to develop TIM-3 antibodies with good activity, wide indications and high yield. It is urgent to develop TIM-3 antibody with better activity to further improve the therapeutic effect. The activity of the antibody itself is affected by the sequences of the variable regions and the structure of the constant region. The sequences of the variable regions of an antibody determine the determinants of antigen recognition, binding affinity, and metabolic rate *in vivo,* which will affect its *in vivo* activity and even the clinical effects of different patients.

At present, the clinical research stage of TIM-3 antibody is mainly used for the treatment of malignant solid tumor and lymphoma, and it is also mainly focused on its combined use with other therapies or target drug and development of antibodies with wide indications so as to expand its applicable clinical symptoms. It is urgent to develop TIM-3 antibodies with higher yield to reduce the treatment cost of patients and benefit more patients. In addition, tumor immunotherapy is expensive, and there is an urgent need to invent and produce new antibodies to reduce costs.

### Summary of the invention

In order to overcome the current lack of fully human TIM-3 antibody and the shortcomings of existing TIM-3 antibodies in terms of activity, the present invention provides a TIM-3 antibody with high affinity and strong specificity, and a preparation method and application thereof.

In a first aspect of the present invention, it provides a heavy chain variable region of an antibody, wherein the heavy chain variable region comprises the following three complementarity determining regions or CDRs:
VH-CDR1 shown in SEQ ID NO. 10n+3,
VH-CDR2 shown in SEQ ID NO. 10n+4, and
VH-CDR3 shown in SEQ ID NO. 10n+5;
wherein each n is independently 0, 1, 2, 3, 4, 5 or 6;
wherein any one of the above amino acid sequences further comprises a derivative sequence which is obtained through optional addition, deletion, modification and/or substitution of at least one amino acid and is capable of retaining TIM-3 binding affinity.

In another preferred embodiment, the heavy chain variable region has the amino acid sequence shown in SEQ ID NO. 10n+1, wherein n is 0, 1, 2, 3, 4, 5 or 6.

In a second aspect of the present invention, it provides a heavy chain of an antibody, which has the heavy chain variable region of the first aspect of the present invention.

In another preferred embodiment, the heavy chain further comprises a heavy chain constant region.

In another preferred embodiment, the heavy chain constant region is of human.

In another preferred embodiment, the heavy chain constant region is a human antibody heavy chain IgG4 constant region.

In a third aspect of the present invention, it provides a light chain variable region of an antibody, wherein the light chain variable region comprises the following three complementarity determining regions or CDRs:
VL-CDR1 shown in SEQ ID NO. 10n+8,
VL-CDR2 shown in SEQ ID NO. 10n+9, and
VL-CDR3 shown in SEQ ID NO. 10n+10;
wherein each n is independently 0, 1, 2, 3, 4, 5 or 6;
wherein any one of the above amino acid sequences further comprises a derivative sequence which is obtained through optional addition, deletion, modification and/or substitution of at least one amino acid and is capable of retaining TIM-3 binding affinity.

In another preferred embodiment, the light chain variable region has the amino acid sequence shown in SEQ ID NO. 10n+6, wherein n is 0, 1, 2, 3, 4, 5 or 6.

In a fourth aspect of the present invention, it provides a light chain of an antibody, which has the light chain variable region of the third aspect of the present invention.

In another preferred embodiment, the light chain further comprises a light chain constant region.

In another preferred embodiment, the light chain constant region is of human.

In another preferred embodiment, the light chain constant region is a human antibody light chain kappa constant region.

In a fifth aspect of the present invention, it provides an antibody having:
(1) the heavy chain variable region of the first aspect of the present invention; and/or
(2) the light chain variable region of the third aspect of the present invention;
alternatively, the antibody has: the heavy chain of the second aspect of the present invention; and/or the light chain of the fourth aspect of the present invention,
wherein any one of the above amino acid sequences further comprises a derivative sequence which is obtained through optional addition, deletion, modification and/or substitution of at least one amino acid and is capable of retaining TIM-3 binding affinity.

In another preferred embodiment, the amino acid sequence of any of the above-mentioned CDRs comprises a derivative CDR sequence with 1, 2 or 3 amino acids added, deleted, modified and/or substituted, and the derivative antibody comprising the VH and VL containing the derivative CDR sequence can retain the binding affinity to TIM-3.

In another preferred embodiment, the ratio (F1/F0) of the binding affinity F1 between the derivatized antibody and TIM-3 to the binding affinity F0 between the corresponding non-derivatized antibody and TIM-3 is 0.5-2, preferably 0.7-1.5, and more preferably 0.8-1.2.

In another preferred embodiment, the number of added, deleted, modified and/or substituted amino acids is 1-5 (such as 1-3, preferably 1-2, more preferably 1).

In another preferred embodiment, the derivative sequence with at least one amino acid added, deleted, modified, and/or substituted, which can retain the binding affinity to TIM-3, is an amino acid sequence having a homology or sequence identity of at least 96%.

In another preferred embodiment, the antibody further comprises a heavy chain constant region and/or a light chain constant region.

In another preferred embodiment, the heavy chain constant region is of human origin, and/or the light chain constant region is of human origin.

In another preferred embodiment, the heavy chain constant region is a human antibody heavy chain IgG4 constant region, and the light chain constant region is a human antibody light chain kappa constant region.

In another preferred embodiment, the antibody is selected from the group consisting of: animal-derived antibodies, chimeric antibodies, humanized antibodies, fully human antibodies, and a combination thereof.

In another preferred embodiment, the ratio (Z1/Z0) of the immunogenicity Z1 of the chimeric antibody in human to the immunogenicity Z0 of the non-chimeric antibody (such as murine-derived antibody) in human is 0-0.5, preferably 0-0.2, more preferably 0-0.05 (e.g. 0.001-0.05).

In another preferred embodiment, the antibody is a partially or fully humanized or fully human monoclonal antibody.

In another preferred embodiment, the antibody is a double chain antibody or a single chain antibody.

In another preferred embodiment, the antibody is a full-length antibody protein or an antigen-binding fragment.

In another preferred embodiment, the antibody is a bispecific antibody or a multispecific antibody.

In another preferred embodiment, the antibody has one or more properties selected from the group consisting of:
(a) inhibiting tumor cell migration or metastasis;
(b) inhibiting tumor growth.

In another preferred embodiment, the antibody comprises the heavy chain variable region according to the first aspect of the present invention and the light chain variable region according to the third aspect of the present invention;
wherein, the heavy chain variable region and the light chain variable region comprise CDRs selected from the following group:

| VH-CDR1 Sequence number | VH-CDR2 Sequence number | VH-CDR3 Sequence number | VL-CDR1 Sequence number | VL-CDR2 Sequence number | VL-CDR3 Sequence number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |

wherein any one of the above amino acid sequences further comprises a derivative sequence which is obtained through optional addition, deletion, modification and/or substitution of at least one amino acid and is capable of retaining TIM-3 binding affinity.

In another preferred embodiment, the antibody comprises the heavy chain variable region according to the first aspect of the present invention and the light chain variable region according to the third aspect of the present invention; wherein,
the heavy chain variable region comprises the following three complementary determining regions or CDRs:
   VH-CDR1 shown in SEQ ID NO. 3,
   VH-CDR2 shown in SEQ ID NO. 4, and
   VH-CDR3 shown in SEQ ID NO. 5;
the light chain variable region comprises the following three complementary determining regions or CDRs:
   VL-CDR1 shown in SEQ ID NO. 8,
   VL-CDR2 shown in SEQ ID NO. 9, and
   VL-CDR3 shown in SEQ ID NO. 10;
   or
the heavy chain variable region comprises the following three complementary determining regions or CDRs:
   VH-CDR1 shown in SEQ ID NO. 13,
   VH-CDR2 shown in SEQ ID NO. 14, and
   VH-CDR3 shown in SEQ ID NO. 15;
the light chain variable region comprises the following three complementary determining regions or CDRs:
   VL-CDR1 shown in SEQ ID NO. 18,
   VL-CDR2 shown in SEQ ID NO. 19, and
   VL-CDR3 shown in SEQ ID NO. 20;
   or
the heavy chain variable region comprises the following three complementary determining regions or CDRs:
   VH-CDR1 shown in SEQ ID NO. 23,
   VH-CDR2 shown in SEQ ID NO. 24, and
   VH-CDR3 shown in SEQ ID NO. 25;
the light chain variable region comprises the following three complementary determining regions or CDRs:
   VL-CDR1 shown in SEQ ID NO. 28,
   VL-CDR2 shown in SEQ ID NO. 29, and
   VL-CDR3 shown in SEQ ID NO. 30;
   or
the heavy chain variable region comprises the following three complementary determining regions or CDRs:
   VH-CDR1 shown in SEQ ID NO. 33,
   VH-CDR2 shown in SEQ ID NO. 34, and
   VH-CDR3 shown in SEQ ID NO. 35;
the light chain variable region comprises the following three complementary determining regions or CDRs:
   VL-CDR1 shown in SEQ ID NO. 38,
   VL-CDR2 shown in SEQ ID NO. 39, and
   VL-CDR3 shown in SEQ ID NO. 40;
   or
the heavy chain variable region comprises the following three complementary determining regions or CDRs:
   VH-CDR1 shown in SEQ ID NO. 43,
   VH-CDR2 shown in SEQ ID NO. 44, and
   VH-CDR3 shown in SEQ ID NO. 45;
the light chain variable region comprises the following three complementary determining regions or CDRs:
   VL-CDR1 shown in SEQ ID NO. 48,
   VL-CDR2 shown in SEQ ID NO. 49, and
   VL-CDR3 shown in SEQ ID NO. 50;
   or
the heavy chain variable region comprises the following three complementary determining regions or CDRs:
   VH-CDR1 shown in SEQ ID NO. 53,
   VH-CDR2 shown in SEQ ID NO. 54, and
   VH-CDR3 shown in SEQ ID NO. 55;
the light chain variable region comprises the following three complementary determining regions or CDRs:
   VL-CDR1 shown in SEQ ID NO. 58,
   VL-CDR2 shown in SEQ ID NO. 59, and
   VL-CDR3 shown in SEQ ID NO. 60;
   or
the heavy chain variable region comprises the following three complementary determining regions or CDRs:
   VH-CDR1 shown in SEQ ID NO. 63,
   VH-CDR2 shown in SEQ ID NO. 64, and
   VH-CDR3 shown in SEQ ID NO. 65;
the light chain variable region comprises the following three complementary determining regions or CDRs:
   VL-CDR1 shown in SEQ ID NO. 68,
   VL-CDR2 shown in SEQ ID NO. 69, and
   VL-CDR3 shown in SEQ ID NO. 70.

In another preferred embodiment, the heavy chain variable region of the antibody has the amino acid sequence shown in SEQ ID NO. 1, 11, 21, 31, 41, 51 or 61; and/or the light chain variable region of the antibody has the amino acid sequence shown in SEQ ID NO. 6, 16, 26, 36, 46, 56 or 66.

In another preferred embodiment, the heavy chain variable region of the antibody has the amino acid sequence shown in SEQ ID NO. 1, and the light chain variable region of the antibody has the amino acid sequence shown in SEQ ID NO. 6.

In another preferred embodiment, the heavy chain variable region of the antibody has the amino acid sequence shown in SEQ ID NO. 11, and the light chain variable region of the antibody has the amino acid sequence shown in SEQ ID NO. 16.

In another preferred embodiment, the heavy chain variable region of the antibody has the amino acid sequence shown in SEQ ID NO. 21, and the light chain variable region of the antibody has the amino acid sequence shown in SEQ ID NO. 26.

In another preferred embodiment, the heavy chain variable region of the antibody has the amino acid sequence shown in SEQ ID NO. 31, and the light chain variable region of the antibody has the amino acid sequence shown in SEQ ID NO. 36.

In another preferred embodiment, the heavy chain variable region of the antibody has the amino acid sequence shown in SEQ ID NO. 41, and the light chain variable region of the antibody has the amino acid sequence shown in SEQ ID NO. 46.

In another preferred embodiment, the heavy chain variable region of the antibody has the amino acid sequence shown in SEQ ID NO. 51, and the light chain variable region of the antibody has the amino acid sequence shown in SEQ ID NO. 56.

In another preferred embodiment, the heavy chain variable region of the antibody has the amino acid sequence shown in SEQ ID NO. 61, and the light chain variable region of the antibody has the amino acid sequence shown in SEQ ID NO. 66.

In another preferred embodiment, the antibody is selected from the group consisting of:

| Antibody number | clone | VH sequence number | VL sequence number |
|---|---|---|---|
| 1 | 7A4F10 | 1 | 6 |
| 2 | 18D2H2 | 11 | 16 |
| 3 | 134H3G6 | 21 | 26 |
| 4 | 215A8F2 | 31 | 36 |
| 5 | 34B6D8 | 41 | 46 |
| 6 | 39E5H1 | 51 | 56 |
| 7 | 57F4E5 | 61 | 66. |

In another preferred embodiment, the amino acid sequence of the heavy chain variable region has a sequence homology or a sequence identity of at least 80%, 85%, 90%, 91%, 92%, 93%, 94%, 95%, 96%, 97%, 98% or 99% with the amino acid sequence shown in SEQ ID NO. 1, 11, 21, 31, 41, 51 or 61 in the sequence listing.

In another preferred embodiment, the amino acid sequence of the light chain variable region has a sequence homology or a sequence identity of at least 80%, 85%, 90%, 91%, 92%, 93%, 94%, 95%, 96%, 97%, 98% or 99% with the amino acid sequence shown in SEQ ID NO. 6, 16, 26, 36, 46, 56 or 66 in the sequence listing.

In a sixth aspect of the present invention, it provides a recombinant protein, wherein the recombinant protein comprises:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(ii) an optional tag sequence that assists expression and/or purification.

In another preferred embodiment, the tag sequence comprises a 6His tag.

In another preferred embodiment, the recombinant protein (or polypeptide) comprises fusion protein.

In another preferred embodiment, the recombinant protein is a monomer, a dimer, or a multimer.

In another preferred embodiment, the recombinant protein comprises
(i) an antibody selected from the group consisting of:

| Antibody number | clone | VH sequence number | VL sequence number |
|---|---|---|---|
| 1 | 7A4F10 | 1 | 6 |
| 2 | 18D2H2 | 11 | 16 |
| 3 | 134H3G6 | 21 | 26 |
| 4 | 215A8F2 | 31 | 36 |
| 5 | 34B6D8 | 41 | 46 |
| 6 | 39E5H1 | 51 | 56 |
| 7 | 57F4E5 | 61 | 66 |

and
(ii) an optional tag sequence to assist expression and/or purification.

In a seventh aspect of the present invention, it provides a polynucleotide, which encodes a polypeptide selected from the group consisting of:
(1) the heavy chain variable region according to the first aspect of the present invention, the heavy chain according to the second aspect of the present invention, the light chain variable region according to the third aspect of the present invention, the light chain according to the fourth aspect of the present invention, or the antibody according to the fifth aspect of the present invention; and
(2) the recombinant protein according to the sixth aspect of the present invention.

In another preferred embodiment, the polynucleotide encoding the heavy chain variable region is as shown in SEQ ID NO. 2, 12, 22, 32, 42, 52 or 62; and/or, the polynucleotide encoding the light chain variable region is as shown in SEQ ID NO. 7, 17, 27, 37, 47, 57 or 67.

In another preferred embodiment, the polynucleotide encoding the heavy chain variable region sequence and the polynucleotide encoding the light chain variable region sequence are selected from the group consisting of:

| Clone | Sequence number of the polynucleotide encoding VH | Sequence number of the polynucleotide encoding VL |
|---|---|---|
| 7A4F10 | 2 | 7 |

| | | |
|---|---|---|
| 18D2H2 | 12 | 17 |
| 134H3G6 | 22 | 27 |
| 215A8F2 | 32 | 37 |
| 34B6D8 | 42 | 47 |
| 39E5H1 | 52 | 57 |
| 57F4E5 | 62 | 67. |

In an eighth aspect of the present invention, it provides a vector, which comprises the polynucleotide according to the seventh aspect of the present invention.

In another preferred embodiment, the vector comprises: a bacterial plasmid, a phage, a yeast plasmid, a plant cell virus, a mammalian cell virus such as an adenovirus, retrovirus, or other vectors.

In a ninth aspect of the present invention, it provides a genetically engineered host cell, wherein the host cell comprises the vector according to the eighth aspect of the present invention or the genome thereof is integrated with the polynucleotide according to the seventh aspect of the present invention.

In a tenth aspect of the present invention, it provides an antibody conjugate, which comprises:
(i) an antibody moiety, which is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, and the antibody according to the fifth aspect of the present invention, and a combination thereof; and
(b) a coupling moiety coupled to the antibody moiety, and the coupling moiety is selected from the group consisting of a detectable label, a drug, a toxin, a cytokine, a radionuclide, an enzyme, and a combination thereof.

In another preferred embodiment, the antibody moiety is coupled to the coupling moiety via a chemical bond or linker.

In an eleventh aspect of the present invention, it provides an immune cell, which expresses or is exposed outside the cell membrane with the antibody according to the fifth aspect of the present invention.

In another preferred embodiment, the immune cell includes NK cells and T cells.

In another preferred embodiment, the immune cell is derived from human or non-human mammals (such as mice).

In a twelfth aspect of the present invention, it provides a pharmaceutical composition, wherein the pharmaceutical composition comprises:
(i) an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, and the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof; and
(ii) a pharmaceutically acceptable carrier.

In another preferred embodiment, the pharmaceutical composition is a liquid formulation.

In another preferred embodiment, the pharmaceutical composition is an injection.

In another preferred embodiment, the pharmaceutical composition comprises 0.01-99.99% of the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof, and 0.01-99.99% of the pharmaceutically acceptable carrier, wherein the percentage is the mass percentage of the pharmaceutical composition.

In a thirteenth aspect of the present invention, it provides use of an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, and the antibody according to the fifth aspect of the present invention, the recombinant protein according to the sixth aspect of the present invention, the antibody conjugate according to the tenth aspect of the present invention, the immune cell according to the eleventh aspect of the present invention, and combinations thereof, wherein the active ingredient is used for (a) preparation of a diagnostic reagent or kit; and/or (b) preparation of a medicine for preventing and/or treating diseases associated with abnormal TIM-3 expression or function.

In another preferred embodiment, the diagnostic reagent is a detection piece or a detection plate.

In another preferred embodiment, the disease associated with abnormal TIM-3 expression or function is selected from the group consisting of tumors and autoimmune diseases.

In another preferred embodiment, the tumor is selected from the group consisting of melanoma, mesothelioma, non-small cell lung cancer, breast cancer, liver cancer, synovial sarcoma, metastatic colon cancer, kidney cancer, bladder cancer, prostate cancer, ovarian cancer, chronic hepatitis C virus infection, advanced solid cancer, malignant tumors of digestive organs, endometrial carcinoma, recurrent melanoma, head and neck squamous cell carcinoma, skin T-cell lymphoma, fallopian tube cancer, peritoneal tumor, muscle invasive bladder cancer, extensive stage small cell lung cancer, adult acute myeloid leukemia, atypical chronic myelogenous leukemia, epithelial ovarian cell carcinoma, B-cell chronic lymphocytic leukemia, skin B-cell non-Hodgkin's lymphoma, intraocular lymphoma, choriocarcinoma of testis, neuroblastoma, esophageal cancer.

In another preferred example, the autoimmune disease is selected from the group consisting of: systemic lupus erythematosus, Ooro-ocular Sjogren's syndrome, rheumatoid arthritis,ankylosing spondylitis, scleroderma, polyarteritis nodosa, Wegener granuloma, hyperthyroidism, insulin-dependent diabetes mellitus, myasthenia gravis, pemphigus vulgaris, pemphigoid, and transplant rejection.

In another preferred embodiment, the diagnostic reagent or kit is used for detecting TIM-3 protein in a sample.

In another preferred embodiment, the diagnostic reagent or kit is used for diagnosis of TIM-3 related diseases.

In another preferred embodiment, the diagnostic reagent or kit is used for detection of TIM-3 protein in a sample.

In a fourteenth aspect of the present invention, it provides a method for *in vitro* detection (including diagnostic or non-diagnostic) of TIM-3 protein in a sample, wherein the method comprises the steps:
(1) contacting the sample with the antibody according to the fifth aspect of the present invention *in vitro;*
(2) detecting whether an antigen-antibody complex is formed, wherein the formation of a complex indicates the presence of TIM-3 protein in the sample.

In a fifteenth aspect of the present invention, it provides a composition for detecting TIM-3 protein in a sample *in vitro,* which comprises the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof, as an active ingredient.

In a sixteenth aspect of the present invention, it provides a detection plate, wherein the detection plate comprises: a substrate (support plate) and a detection strip, wherein the detection strip comprises the antibody according to the fifth aspect of the present invention, the recombinant protein according to the sixth aspect of the present invention, the antibody conjugate according to the tenth aspect of the present invention, the immune cell according to the eleventh aspect of the present invention, or a combination thereof.

In a seventeenth aspect of the present invention, it provides a kit, which comprises:
(1) a first container, which contains the antibody of the present invention; and/or
(2) a second container, which contains a secondary antibody against the antibody of the present invention;
or,
the kit comprises the detection plate according to the sixteenth aspect of the present invention.

In an eighteenth aspect of the present invention, it provides a method for preparing a recombinant polypeptide, wherein the method comprises:
(a) culturing the host cell according to the ninth aspect of the present invention under conditions suitable for expression;
(b) isolating a recombinant polypeptide from the culture, wherein the recombinant polypeptide is the antibody according to the fifth aspect of the present invention or the recombinant protein according to the sixth aspect of the present invention.

In a nineteenth aspect of the present invention, it provides a drug combination, comprising:
(i) a first active ingredient, which comprises the antibody according to the fifth aspect of the present invention, or the recombinant protein according to the sixth aspect of the present invention, or the antibody conjugate according to the tenth aspect of the present invention, or the immune cell according to the eleventh aspect of the present invention, or the pharmaceutical composition according to the twelfth aspect of the present invention, or a combination thereof;
(ii) a second active ingredient, which comprises a second antibody, or a chemotherapeutic agent.

In another preferred embodiment, the second antibody is selected from the group consisting of a CTLA4 antibody and a PD-1 antibody.

In another preferred embodiment, the second antibody is a PD-1 antibody.

In another preferred example, the chemotherapeutic agent is selected from the group consisting of docetaxel, carboplatin, and a combination thereof.

In a twentieth aspect of the present invention, it provides use of the antibody according to the fifth aspect of the present invention, or the recombinant protein according to the sixth aspect of the present invention, or the antibody conjugate according to the tenth aspect of the present invention, or the immune cell according to the eleventh aspect of the present invention, and/or the pharmaceutical composition according to the twelfth aspect of the present invention, in combination with a second antibody or a chemotherapeutic agent, for preparation of a medicine for the treatment of diseases associated with abnormal TIM-3 expression or function.

In another preferred embodiment, the second antibody is selected from the group consisting of a CTLA4 antibody and a PD-1 antibody.

In another preferred embodiment, the second antibody is a PD-1 antibody.

In a twenty-first aspect of the present invention, it provides a method for the treatment of diseases associated with abnormal TIM-3 expression or function, comprising administering an effective amount of the antibody according to the fifth aspect of the present invention, the recombinant protein according to the sixth aspect of the present invention, the antibody conjugate according to the tenth aspect of the present invention, the immune cell according to the eleventh aspect of the present invention, the pharmaceutical composition of the twelfth aspect of the present invention, or a combination thereof, to a subject in need.

In another preferred embodiment, the disease associated with abnormal TIM-3 expression or function is cancer.

In another preferred embodiment, the method further comprises: administering a safe and effective amount of a second antibody to the subject before, during, and/or after administering the first active ingredient.

In another preferred embodiment, the second antibody is selected from the group consisting of a PD-1 antibody and a CTLA4 antibody.

In another preferred embodiment, the second antibody is a PD-1 antibody.

It should be understood that within the scope of the present invention, the various technical features of the present invention above and the various technical features specifically described hereinafter (as in the embodiments) may be combined with each other to constitute a new or preferred technical solution, which needs not be described one by one, due to space limitations.

### Description of the figures

Figure 1 shows the binding activity of TIM-3-hFc protein to its commercial antibody.
Figure 2 shows the FACS detection results of HEK293 cells transfected with TIM-3 gene.
Figure 3 shows the ELISA detection of the serum antibody titer of mice immunized with TIM-3-hFC protein.
Figure 4 shows the activity of TIM-3 antibodies reacting with human TIM-3 extracellular domain protein in the enzyme-linked immunosorbent assay.
Figure 5 shows the activity of TIM-3 antibodies reacting with monkey TIM-3 extracellular domain protein in the enzyme-linked immunosorbent assay.
Figure 6 shows the activity of TIM-3 antibodies reacting with mouse TIM-3 extracellular domain protein in the enzyme-linked immunosorbent assay.
Figure 7 shows the FACS detection of the binding reaction of TIM-3 antibodies to CHOK1-hTIM-3.
Figure 8 shows the FACS detection of the binding reaction of TIM-3 antibodies to CHOK1-cTIM-3.
Figure 9 shows the effects of the antibodies on IFN-γ secretion in the OKT3-dependent PBMC activation assay.
Figure 10 shows the activity of fully human TIM-3 antibodies reacting with human TIM-3 extracellular domain protein in the enzyme-linked immunosorbent assay.
Figure 11 shows the activity of fully human TIM-3 antibodies reacting with monkey TIM-3 extracellular domain protein in the enzyme-linked immunosorbent assay.
Figure 12 shows the activity of fully human TIM-3 antibodies reacting with mouse TIM-3 extracellular domain protein in the enzyme-linked immunosorbent assay.
Figure 13 shows the FACS detection of the binding reaction of fully human TIM-3 antibodies to CHOK1-hTIM-3.
Figure 14 shows the FACS detection of the binding reaction of fully human TIM-3 antibodies to CHOK1-cTIM-3.
Figure 15 shows that the fully human TIM-3 antibodies block the binding of TIM-3 protein to its receptor phosphatidylserine.
Figure 16 shows the effect of fully human TIM-3 antibodies on IFN-γ secretion in lymphocyte activation assay (donor X).
Figure 17 shows the effect of fully human TIM-3 antibodies on IFN-γ secretion in lymphocyte activation assay (donor Y).

### Modes for carrying out the present invention

Through extensive and intensive studies, the inventors prepared TIM-3 antibodies by optimized hybridoma technology using human TIM-3 as immunogen. Specifically, the invention prepared fully human antibody using human antibody transgenic mouse technology and obtained leading antibody of the TIM-3 antibody. Then, through the preliminary production, purification and verification of the leading antibody, TIM-3 antibody with excellent biological characteristics such as high antibody affinity and significantly increasing IFN expression level in the activation reaction of human peripheral blood mononuclear cells was obtained. Then the amino acid sequences of heavy chain variable region and light chain variable region of the TIM-3 antibody were obtained by molecular biology sequencing. The TIM-3 antibody has high affinity with TIM-3 proteins of human origin and monkey origin, and can increase the expression level of IFN in human peripheral blood mononuclear cells induced by OKT3 activation. The present invention also provides uses of these antibodies, including but not limited to inhibiting the negative regulation of signaling pathways mediated by TIM-3 and its ligand, activating tumor-specific immune responses, and being used alone or in combination with anti-PD-1, CTLA-4 monoclonal antibodies or other anti-tumor drugs for tumor immunotherapy. The present invention has been completed on the basis of this.

### Terms

In the present invention, "VH-CDR1" and "CDR-H1" can be used interchangeably and both refer to CDR1 of heavy chain variable region; "VH-CDR2" and "CDR-H2" can be used interchangeably and both refer to CDR2 of heavy chain variable region; "VH-CDR3" and "CDR-H3" can be used interchangeably and both refer to CDR3 of heavy chain variable region. "VL-CDR1" and "CDR-L1" can be used interchangeably and both refer to CDR1 of light chain variable region; "VL-CDR2" and "CDR-L2" can be used interchangeably and both refer to CDR2 of light chain variable region; "VL-CDR3" and "CDR-L3" can be used interchangeably and both refer to CDR3 of light chain variable region.

### Antibody

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of a light chain pairs with the first constant region of a heavy chain, and the variable region of a light chain pairs with the variable region of a heavy chain. Special amino acid residues form an interface between the variable regions of a light chain and a heavy chain.

As used herein, the term "variable" means that antibodies are different from each other in terms of sequence in certain parts of variable regions, which is responsible for the binding and specificity of various specific antibodies to their specific antigens. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). The variable regions of the natural heavy and light chains each contain four FR regions, which are roughly in the β-folded configuration, connected by the three CDRs that form the connecting loop, and in some cases may form a partly β folded structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of an antibody to an antigen, however, they exhibit different effector functions, such as involved in the antibody-dependent cytotoxicity of an antibody.

The light chains of vertebrate antibodies (immunoglobulins) can be classified into one of two distinct classes (referred to as κ and λ) based on the amino acid sequence of their constant regions. Immunoglobulins can be classified into different classes depending on the amino acid sequences of their heavy chain constant regions. There are mainly five classes of immunoglobulins: IgA, IgD, IgE, IgG, and IgM, some of which can be further classified into subclasses (isotypes), such as IgG1, IgG2, IgG3, IgG4, IgA, and IgA2. The heavy chain constant regions corresponding to different classes of immunoglobulins are called α, δ, ε, γ, and µ, respectively. The subunit structures and three-dimensional configurations of different classes of immunoglobulins are well known for those skilled in the art.

In general, the antigen binding characteristics of an antibody can be described by three specific regions located in the heavy and light chain variable regions, called complementarity determining regions (CDRs), which divide the variable region into four framework regions (FRs); the amino acid sequences of the four FRs are relatively conservative and are not directly involved in the binding reaction. These CDRs form a ring structure, and approach to each other in the steric structure by virtue of the β-sheets formed by the FRs between them, and the CDRs on the heavy chain and the CDRs on the corresponding light chain constitute the antigen-binding site of an antibody. By comparison of the amino acid sequences of antibodies of the same type, it can be determined which amino acids form FRs or CDRs.

The present invention includes not only an intact antibody, but also the fragments of the antibody having an immunological activity or a fusion protein formed by the antibody and another sequence. Therefore, the present invention also includes fragments, derivatives and analogs of the antibody.

In the present invention, antibodies include murine, chimeric, humanized or fully human antibodies as prepared by techniques well known to those skilled in the art. Recombinant antibodies, such as chimeric and humanized monoclonal antibodies, including human and non-human portions, can be obtained by standard DNA recombination techniques, all of which are useful antibodies. A chimeric antibody is a molecule in which different portions are derived from different animal species, for example, a chimeric antibody having a variable region from a monoclonal antibody from a mouse and a constant region from a human immunoglobulin (see, for example, U.S. Pat. Nos. 4,816,567 and 4,816,397, which are incorporated herein by reference in its entirety). A humanized antibody refers to an antibody molecule derived from a non-human species, which has one or more complementarity determining regions (CDRs) derived from a non-human species and framework regions derived from a human immunoglobulin molecule (see U.S. Pat. No. 5,585,089, which is incorporated herein by reference in its entirety). These chimeric and humanized monoclonal antibodies can be prepared by recombinant DNA techniques well known in the art.

In the present invention, an antibody may be monospecific, bispecific, trispecific, or multispecific.

In the present invention, the antibody of the present invention further includes a conservative variant thereof, which refers to a polypeptide formed by substitution of at most 10, preferably at most 8, more preferably at most 5, and most preferably at most 3 amino acids with amino acids having similar or analogous property, as compared to the amino acid sequence of the antibody of the present invention. These conservatively variant polypeptides are preferably produced by amino acid substitution according to Table 19.

**Table 19**

| Initial residue | Representative substitution | Preferred sub stitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

| | | |
|---|---|---|
| **Anti-TIM-3 antibody** | | |

In the present invention, the antibody is an anti-TIM-3 antibody. The present invention provides an antibody with high specificity and high affinity against TIM-3, which comprises a heavy chain and a light chain, wherein the heavy chain contains a heavy chain variable region (VH) amino acid sequence, and the light chain contains a light chain variable region (VL) amino acid sequence.

Preferably,
the heavy chain variable region (VH) has complementarity determining regions or CDRs selected from the group consisting of:
   VH-CDR1 shown in SEQ ID NO. 10n+3,
   VH-CDR2 shown in SEQ ID NO. 10n+4, and
   VH-CDR3 shown in SEQ ID NO. 10n+5;
   wherein each n is independently 0, 1, 2, 3, 4, 5 or 6;
the light chain variable region (VL) has complementarity determining regions or CDRs selected from the group consisting of:
   VL-CDR1 shown in SEQ ID NO. 10n+8,
   VL-CDR2 shown in SEQ ID NO. 10n+9, and
   VL-CDR3 shown in SEQ ID NO. 10n+10;
   wherein each n is independently 0, 1, 2, 3, 4, 5 or 6;
wherein any one of the above amino acid sequences further comprises a derivative sequence which is obtained through optional addition, deletion, modification and/or substitution of at least one amino acid and is capable of retaining TIM-3 binding affinity.

Preferably, the heavy chain variable region (VH) comprises the following three complementary determining regions or CDRs:
VH-CDR1 shown in SEQ ID NO. 10n+3,
VH-CDR2 shown in SEQ ID NO. 10n+4, and
VH-CDR3 shown in SEQ ID NO. 10n+5;
the light chain variable region (VL) comprises the following three complementary determining regions or CDRs:
VL-CDR1 shown in SEQ ID NO. 10n+8,
VL-CDR2 shown in SEQ ID NO. 10n+9, and
VL-CDR3 shown in SEQ ID NO. 10n+10;
wherein each n is independently 0, 1, 2, 3, 4, 5 or 6; preferably n is 0 or 1;
wherein any one of the above amino acid sequences further comprises a derivative sequence which is obtained through optional addition, deletion, modification and/or substitution of at least one amino acid and is capable of retaining the binding affinity to TIM-3.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

Method for determining sequence homology or identity that are well known to the ordinary skilled in the art includes, but are not limited to: Computer Molecular Biology, edited by Lesk, A.M., Oxford University Press, New York, 1988; Biocomputing; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987, and Sequence Analysis Primer, edited by Gribskov, M. and Devereux, J., M Stockton Press, New York, 1991 and Carillo, H. & Lipman, D., SIAM J. Applied Math ., 48:1073(1988). The preferred method for determining identity is to obtain the greatest match between the sequences tested. Methods for determining identity are compiled into publicly available computer programs. Preferred computer program method for determining identity between two sequences includes, but are not limited to, the GCG software package (Devereux, J. et al., 1984), BLASTP, BLASTN, and FASTA (Altschul, S, F. et al., 1990). The BLASTX program is available to the public from NCBI and other sources (BLAST Handbook, Altschul, S. et al., NCBI NLM NIH Bethesda, Md. 20894; Altschul, S. et al., 1990). The well-known Smith Waterman algorithm can also be used to determine identity.

The antibody in the present invention can be a full-length protein (such as IgG1, IgG2a, IgG2b or IgG2c), or a protein fragment containing an antigen-antibody binding domain (such as Fab, F(ab'), sdAb, ScFv fragments). The antibody in the present invention can be a wild-type protein, or a mutant protein that has achieved a certain effect through specific mutations, for example, using mutations to eliminate the effector function of the antibody.

Preferably, the antibody described herein is one or more of an antibody full-length protein, an antigen-antibody binding domain protein fragment, a bispecific antibody, a multispecific antibody, a single chain antibody fragment (scFv), a single domain antibody (sdAb) and a single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully human antibody full-length protein. Preferably, the antibody full-length protein is IgG1, IgG2, IgG3 or IgG4.

The antibody of the present invention may be a double-chain or single-chain antibody, and may be selected from animal-derived antibodies, chimeric antibodies and humanized antibodies, more preferably be selected from humanized antibodies and human-animal chimeric antibodies, more preferably a fully humanized antibody.

The antibody derivative of the present invention may be a single-chain antibody, and/or an antibody fragment, for example, Fab, Fab', (Fab')2 or other antibody derivatives known in the art, etc., and may be any one or more of IgA, IgD, IgE, IgG and IgM antibodies or other subtype antibodies.

The single-chain antibody is a conventional single-chain antibody in the art, which comprises a heavy chain variable region, a light chain variable region and a short peptide of 15-20 amino acids.

In the present invention, the animal is preferably a mammal, such as mouse.

The antibody of the present invention may be a chimeric antibody, a humanized antibody, a CDR grafted and/or modified antibody that targets TIM-3, such as human TIM-3.

In above content of the present invention, the number of the added, deleted, modified and/or substituted amino acids, is preferably not more than 40%, more preferably not more than 35%, more preferably 1-33%, more preferably 5-30%, more preferably 10-25%, and more preferably 15-20% of the total number of the amino acids of the initial amino acid sequence.

In the above content of the present invention, more preferably, the number of the added, deleted, modified and/or substituted amino acids, may be 1-7, more preferably 1-5, more preferably 1-3, and more preferably 1-2.

In another preferred embodiment, the heavy chain variable region of the antibody has the amino acid sequence shown in SEQ ID NO. 1, 11, 21, 31, 41, 51 or 61.

In another preferred embodiment, the light chain variable region of the antibody has the amino acid sequence shown in SEQ ID NO. 6, 16, 26, 36, 46, 56 or 66.

In another preferred embodiment, the amino acid sequences of the heavy chain variable region and/or the light chain variable region of the antibody targeting TIM-3 are shown in the following Table 20:

**Table 20**

| Antibody number | VH sequence number | VL sequence number |
|---|---|---|
| 1 | 1 | 6 |
| 2 | 11 | 16 |
| 3 | 21 | 26 |
| 4 | 31 | 36 |
| 5 | 41 | 46 |
| 6 | 51 | 56 |
| 7 | 61 | 66 |

In another preferred embodiment, the antibody targeting TIM-3 is 7A4F10, 10D2H2, 134H3G6, 215A8F2, 34B6D8, 39E5H1 or 57F4E5.

In another preferred embodiment, the antibody targeting TIM-3 is 7A4F10.

In another preferred embodiment, the antibody targeting TIM-3 is 10D2H2.

### Recombinant protein

The invention also provides a recombinant protein comprising one or more of heavy chain CDR1 (VH-CDR1), heavy chain CDR2 (VH-CDR2) and heavy chain CDR3 (VH-CDR3) of a TIM-3 antibody, and/or one or more of light chain CDR1 (VL-CDR1), light chain CDR2 (VL-CDR2) and light chain CDR3 (VL-CDR3) of a TIM-3 antibody,

The sequences of the heavy chain CDR1-3 are as follows:
VH-CDR1 shown in SEQ ID NO. 10n+3,
VH-CDR2 shown in SEQ ID NO. 10n+4,
VH-CDR3 shown in SEQ ID NO. 10n+5;

The sequences of the light chain CDR1-3 are as follows:
VL-CDR1 shown in SEQ ID NO. 10n+8,
VL-CDR2 shown in SEQ ID NO. 10n+9, and
VL-CDR3 shown in SEQ ID NO. 10n+10;
wherein each n is independently 0, 1, 2, 3, 4, 5 or 6; preferably n is 0 or 1;
wherein any one of the above amino acid sequences further comprises a derivative sequence which is obtained through optional addition, deletion, modification and/or substitution of at least one amino acid and is capable of retaining the binding affinity to TIM-3.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

In another preferred embodiment, the recombinant protein according to the present invention comprises a heavy chain variable region of a TIM-3 antibody and/or a light chain variable region of a TIM-3 antibody, the heavy chain variable region of the antibody has the amino acid sequence shown in SEQ ID NO. 1, 11, 21, 31, 41, 51 or 61; and the light chain variable region of the antibody has the amino acid sequence shown in SEQ ID NO. 6, 16, 26, 36, 46, 56 or 66.

In another preferred embodiment, the recombinant protein according to the present invention comprises a heavy chain variable region of a TIM-3 antibody and a light chain variable region of a TIM-3 antibody, the heavy chain variable region of the antibody has the amino acid sequence shown in SEQ ID NO. 1, 11, 21, 31, 41, 51 or 61; and the light chain variable region of the antibody has the amino acid sequence shown in SEQ ID NO. 6, 16, 26, 36, 46, 56 or 66.

In another preferred embodiment, the amino acid sequence numbers of the recombinant protein as well as the heavy chain CDR1-3 and light chain CDR1-3 comprised therein are as shown in Table 21:

**Table 21 Amino acid sequence numbers of heavy chain CDR1-3 and light chain CDR1-3**

| Recombi nant protein numbers | Heavy chain protein | | | | Light chain protein | | | |
|---|---|---|---|---|---|---|---|---|
| | Variable region | VH-CDR 1 | VH-CDR 2 | VH-CDR 3 | Variable region | VL-CDR 1 | VL-CDR 2 | VL-CDR 3 |
| 1 | 1 | 3 | 4 | 5 | 6 | 8 | 9 | 10 |
| 2 | 11 | 13 | 14 | 15 | 16 | 18 | 19 | 20 |
| 3 | 21 | 23 | 24 | 25 | 26 | 28 | 29 | 30 |
| 4 | 31 | 33 | 34 | 35 | 36 | 38 | 39 | 40 |
| 5 | 41 | 43 | 44 | 45 | 46 | 48 | 49 | 50 |
| 6 | 51 | 53 | 54 | 55 | 56 | 58 | 59 | 60 |
| 7 | 61 | 63 | 64 | 65 | 66 | 68 | 69 | 70 |

wherein any one of the above amino acid sequences further comprises a derivative sequence which is obtained through optional addition, deletion, modification and/or substitution of at least one amino acid and is capable of retaining the binding affinity to TIM-3.

Preferably, the recombinant protein further comprises an antibody heavy chain constant region and/or an antibody light chain constant region, wherein the antibody heavy chain constant region is conventional in the art, preferably a rat antibody heavy chain constant region or a human antibody heavy chain constant region, more preferably a human antibody heavy chain constant region. The antibody light chain constant region is conventional in the art, preferably a rat antibody light chain constant region or a human antibody light chain constant region, more preferably a human antibody light chain constant region.

The recombinant protein is a conventional protein in the art. Preferably, it is one or more of an antibody full-length protein, an antigen-antibody binding domain protein fragment, a bispecific antibody, a multispecific antibody, a single chain antibody fragment (scFv), a single domain antibody (sdAb) and a single-domain antibody, as well as a monoclonal antibody or a polyclonal antibody made from the above antibodies. The monoclonal antibody can be developed by a variety of approaches and technologies, including hybridoma technology, phage display technology, single lymphocyte gene cloning technology, etc. The mainstream is to prepare monoclonal antibodies from wild-type or transgenic mice through hybridoma technology.

The antibody full-length protein is a conventional antibody full-length protein in the art, which comprises a heavy chain variable region, a light chain variable region, a heavy chain constant region, and a light chain constant region. The heavy chain variable region and light chain variable region of the protein and human heavy chain constant region and human light chain constant region constitute a fully human antibody full-length protein. Preferably, the antibody full-length protein is IgG1, IgG2, IgG3 or IgG4.

The single-chain antibody is a conventional single-chain antibody in the art, which comprises a heavy chain variable region, a light chain variable region and a short peptide of 15-20 amino acids.

The antigen-antibody binding domain protein fragments are conventional antigen-antibody binding domain protein fragments in the art, which comprise a light chain variable region, a light chain constant region, and an Fd segment of heavy chain constant region. Preferably, the antigen-antibody binding domain protein fragments are Fab and F (ab').

The single domain antibody is a conventional single domain antibody in the art, which comprises a heavy chain variable region and a heavy chain constant region.

The single-domain antibody is a conventional single-domain antibody in the art, which only comprises a heavy chain variable region.

Wherein, the preparation method of the recombinant protein is a conventional preparation method in the art. Preferably, the preparation method is: isolating and obtaining the protein from an expression transformant that recombinantly expresses the protein or obtaining the protein by artificially synthesizing a protein sequence. The method of isolating and obtaining the protein from an expression transformant that recombinantly expresses the protein is preferably as follows: cloning a nucleic acid molecule encoding the protein carrying a point mutation into a recombinant vector, and transforming the obtained recombinant vector into a transformant to obtain a recombinant expression transformant, and by culturing the obtained recombinant expression transformant, the recombinant protein can be obtained by separation and purification.

### Nucleic Acid

The present invention also provides a nucleic acid encoding the above-mentioned antibody (e.g., an anti-TIM-3 antibody) or recombinant protein, or the heavy chain variable region or the light chain variable region of the anti-TIM-3 antibody.

Preferably, the nucleotide sequence of the nucleic acid encoding the heavy chain variable region is shown in SEQ ID NO. 2, 12, 22, 32, 42, 52 or 62 in the sequence listing; and/or, the nucleotide sequence of the nucleic acid encoding the light chain variable region is shown in SEQ ID NO. 7, 17, 27, 37, 47, 57 or 67 in the sequence listing.

More preferably, the nucleotide sequence of the nucleic acid encoding the heavy chain variable region is shown in SEQ ID NO. 2, 12, 22, 32, 42, 52 or 62 in the sequence listing; and, the nucleotide sequence of the nucleic acid encoding the light chain variable region is shown in SEQ ID NO. 7, 17, 27, 37, 47, 57 or 67 in the sequence listing.

The preparation method of the nucleic acid is a conventional preparation method in the art. Preferably, it comprises the following steps: obtaining the nucleic acid molecule encoding the above-mentioned protein by gene cloning technology, or obtaining the nucleic acid molecule encoding the above-mentioned protein by the method of artificial full-length sequence synthesis.

Those skilled in the art know that the base sequence encoding the amino acid sequence of the protein can be replaced, deleted, changed, inserted or added appropriately to provide a polynucleotide homolog. The homolog of the polynucleotide of the present invention can be prepared by replacing, deleting or adding one or more bases of the gene encoding the protein sequence within the scope of maintaining the activity of the antibody.

### Vector

The present invention also provides a recombinant expression vector comprising the nucleic acid.

The recombinant expression vector can be obtained by conventional methods in the art, that is, by connecting the nucleic acid molecule of the present invention to various expression vectors, thus being constructed. The expression vector is one of a variety of conventional vectors in the art, as long as it can carry the above-mentioned nucleic acid molecule. The vector preferably includes: various plasmids, cosmids, phage or virus vectors and the like.

The present invention also provides a recombinant expression transformant comprising the above-mentioned recombinant expression vector.

Wherein, the preparation method of the recombinant expression transformant is a conventional preparation method in the art, preferably comprising: being obtained by transforming the recombinant expression vector into a host cell. The host cell is one of a variety of conventional host cells in the art, as long as the recombinant expression vector can replicate itself stably and the nucleic acid carried can be effectively expressed. Preferably, the host cell is E.coli TG1 or E.coli BL21 cell (for expressing single-chain antibodies or Fab antibodies), or HEK293 or CHO cell (for expressing full-length IgG antibodies). The above-mentioned recombinant expression plasmid is transformed into a host cell to obtain the preferred recombinant expression transformant of the present invention. The transformation method is a conventional transformation method in the art, preferably a chemical transformation method, a heat shock method or an electrotransformation method.

### Antibody preparation

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

At present, it is possible to obtain a DNA sequence encoding the antibody of the present invention (or fragments thereof, or derivatives thereof) completely by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody of the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. The examples of these methods comprise, but are not limited to, conventional renaturation treatment, treatment by protein precipitant (such as salt precipitation), centrifugation, cell lysis by osmosis, ultrasonic treatment, supercentrifugation, molecular sieve chromatography (gel chromatography), adsorption chromatography, ion exchange chromatography, high performance liquid chromatography (HPLC), and any other liquid chromatography, and the combination thereof.

### Antibody-drug conjugate (ADC)

The present invention also provides an antibody-drug conjugate (ADC) based on the antibody according to the present invention.

Typically, the antibody-drug conjugate comprises the antibody and an effector molecule, wherein the antibody is conjugated to the effector molecule, and chemical conjugation is preferred. Preferably, the effector molecule is a therapeutically active drug. In addition, the effector molecule may be one or more of a toxic protein, a chemotherapeutic drug, a small-molecule drug or a radionuclide.

The antibody according to present invention and the effector molecule may be coupled by a coupling agent. Examples of the coupling agent may be any one or more of a non-selective coupling agent, a coupling agent utilizing a carboxyl group, a peptide chain, and a coupling agent utilizing a disulfide bond. The non-selective coupling agent refers to a compound that results in a linkage between an effector molecule and an antibody via a covalent bond, such as glutaraldehyde, etc. The coupling agent utilizing a carboxyl group may be any one or more of cis-aconitic anhydride coupling agents (such as cis-aconitic anhydride) and acyl hydrazone coupling agents (the coupling site is acyl hydrazone).

Certain residues on an antibody (such as Cys or Lys, etc.) are used to link a variety of functional groups, including imaging agents (such as chromophores and fluorophores), diagnostic agents (such as MRI contrast agents and radioisotopes), stabilizers (such as poly(ethylene glycol)) and therapeutic agents. An antibody can be conjugated to a functional agent to form a conjugate of the antibody-functional agent. A functional agent (e.g. a drug, a detection reagent, a stabilizer) is conjugated (covalently linked) to an antibody. A functional agent can be linked to an antibody either directly or indirectly via a linker.

Antibodies can be conjugated to drugs to form antibody-drug conjugates (ADCs). Typically, an ADC comprises a linker between a drug and an antibody. The linker can be a degradable or non-degradable linker. Typically, degradable linkers are easily degraded in an intracellular environment, for example, the linker is degraded at the target site, thereby releasing the drug from the antibody. Suitable degradable linkers include, for example, enzyme-degradable linkers, including peptidyl-containing linkers that can be degraded by protease (e.g. lysosomal protease or endosomal protease) in a cell, or sugar linkers, for example, glucuronide-containing linkers that can be degraded by glucuronidase. Peptidyl linkers may include, for example, dipeptides, such as valine-citrulline, phenylalanine-lysine or valine-alanine. Other suitable degradable linkers include, for example, pH sensitive linkers (e.g. linkers that are hydrolyzed at a pH of below 5.5, such as hydrazone linkers) and linkers that are degraded under reducing conditions (e.g. disulfide-bond linkers). A non-degradable linker typically releases a drug under conditions that the antibody is hydrolyzed by protease.

Prior to linkage to an antibody, a linker has a reactive group capable of reacting with certain amino acid residues, and the linkage is achieved by the reactive group. A thiol-specific reactive group is preferred, and includes, for example, a maleimide compound, a halogenated (e.g. iodo-, bromo- or chloro-substituted) amide; a halogenated (e.g. iodo-, bromo- or chloro-substituted) ester; a halogenated (e.g. iodo-, bromo- or chloro-substituted) methyl ketone, a benzyl halide (e.g. iodide, bromide or chloride); vinyl sulfone, pyridyl disulfide; a mercury derivative such as 3,6-di-(mercurymethyl)dioxane, wherein the counter ion is CH₃COO⁻, Cl⁻ or NO₃⁻; and polymethylene dimethyl sulfide thiosulfonate. The linker may include, for example, a maleimide linked to an antibody via thiosuccimide.

A drug may be any cytotoxic drug which inhibits cell growth or immunosuppression. In an embodiment, an antibody is linked to a drug via a linker, and the drug has a functional group that can form a bond with the linker. For example, a drug may have an amino group, a carboxyl group, a thiol group, a hydroxyl group, or a ketone group that can form a bond with a linker. When a drug is directly linked to a linker, the drug has a reactive group before being linked to an antibody.

Useful drugs include, for example, anti-tubulin drugs, DNA minor groove binding agents, DNA replication inhibitors, alkylating agents, antibiotics, folic acid antagonists, antimetabolites, chemotherapy sensitizers, topoisomerase inhibitors, vinca alkaloids, etc. Examples of particularly useful cytotoxic drugs include, for example, DNA minor groove binding agents, DNA alkylating agents, and tubulin inhibitors; typical cytotoxic drugs include, for example, auristatins, camptothecins, docamycin/duocarmycins, etoposides, maytansines and maytansinoids (e.g. DM1 and DM4), taxanes, benzodiazepines or benzodiazepine containing drugs (e.g. pyrrolo[1,4]benzodiazepines (PBDs), indolinobenzodiazepines and oxazolidinobenzodiazepines), and vinca alkaloids.

In the present invention, a drug-linker can be used to form an ADC in a simple step. In other embodiments, a bifunctional linker compound can be used to form an ADC in a two-step or multi-step process. For example, a cysteine residue is reacted with the reactive moiety of a linker in a first step, and then the functional group on the linker is reacted with a drug in the subsequent step, so as to form an ADC.

In general, the functional group on a linker is selected so that it can specifically react with the suitable reactive group on a drug moiety. As a non-limiting example, an azide-based moiety can be used to specifically react with the reactive alkynyl group on a drug moiety. The drug is covalently bound to the linker by 1,3-dipolar cycloaddition between the azide and alkynyl group. Other useful functional groups include, for example, ketones and aldehydes (suitable for reacting with hydrazides and alkoxyamines), phosphines (suitable for reacting with azides); isocyanates and isothiocyanates (suitable for reacting with amines and alcohols); and activated esters, for example, N-hydroxysuccinimide esters (suitable for reacting with amines and alcohols). These and other linkage strategies, for example, those described in "Bioconjugation Technology" (2nd Edition (Elsevier)), are well known to those skilled in the art. Those skilled in the art could understand that when a complementary pair of reactive functional groups are selected for a selective reaction between a drug moiety and a linker, each member of the complementary pair can be used for the linker, and can also be used for the drug.

The present invention further provides a method for preparing an ADC, which may further comprise: under conditions sufficient to form an antibody-drug conjugate (ADC), binding an antibody to a drug-linker compound.

In certain embodiments, the method according to the present invention comprises: under conditions sufficient to form an antibody-linker conjugate, binding an antibody to a bifunctional linker compound. In these embodiments, the method according to the present invention further comprises: under conditions sufficient to covalently link the drug moiety to the antibody via a linker, binding the antibody-linker conjugate to the drug moiety.

In some embodiments, an antibody-drug conjugate (ADC) has a formula as follows: 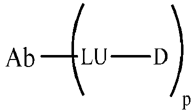 wherein:
Ab is an antibody,
LU is a linker;
D is a drug;
And the subscript p is a value selected from 1 to 8.

### Applications

The present invention also provides use of the antibody, the antibody conjugate ADC, the recombinant protein, and/or immune cell of the present invention, for example for the preparation of diagnostic preparations or the preparation of drugs.

Preferably, the drug is for prevention and/or treatment of diseases associated with abnormal TIM-3 expression or function.

Uses of the antibody, the ADC, the recombinant protein, and/or the immune cell of the present invention include (but are not limited to):
(i) diagnosis, prevention and/or treatment of tumorigenesis, for tumor growth and/or metastasis, particularly, for a tumor with TIM-3 high expression; wherein the tumor includes (but not limited to): melanoma, mesothelioma, non-small cell lung cancer, breast cancer, liver cancer, synovial sarcoma, metastatic colon cancer, kidney cancer, bladder cancer, prostate cancer, ovarian cancer, chronic hepatitis C virus infection, advanced solid cancer, malignant tumors of digestive organs, endometrial carcinoma, recurrent melanoma, head and neck squamous cell carcinoma, skin T-cell lymphoma, fallopian tube cancer, peritoneal tumor, muscle invasive bladder cancer, extensive stage small cell lung cancer, adult acute myeloid leukemia, atypical chronic myelogenous leukemia, epithelial ovarian cell carcinoma, B-cell chronic lymphocytic leukemia, skin B-cell non-Hodgkin's lymphoma, intraocular lymphoma, choriocarcinoma of testis, neuroblastoma, esophageal cancer;
(ii) diagnosis, prevention and/or treatment of an autoimmune diseases, wherein the autoimmune disease includes (but not limited to): systemic lupus erythematosus, Ooro-ocular Sjogren's syndrome, rheumatoid arthritis,ankylosing spondylitis, scleroderma, polyarteritis nodosa, Wegener granuloma, hyperthyroidism, insulin-dependent diabetes mellitus, myasthenia gravis, pemphigus vulgaris, pemphigoid, and transplant rejection.

### Use for detection and kits

The antibody or ADC thereof of the present invention can be used for detection, for example, for detecting samples, thereby providing diagnostic information.

In the present invention, the samples (specimens) used include cells, tissue samples and biopsy specimens. The term "biopsy" used in the present invention shall include all kinds of biopsy known to those skilled in the art. Therefore, the biopsy used in the present invention may include, for example, excision samples of tumors, tissue samples prepared by endoscopic methods or organ puncture or needle biopsy.

The samples used in the present invention include fixed or preserved cell or tissue samples.

The present invention also provides a kit comprising the antibody (or fragment thereof) of the present invention. In a preferred embodiment of the present invention, the kit further includes a container, an instruction for use, buffer, and the like. In a preferred embodiment, the antibody of the present invention can be immobilized on a detection plate.

### Pharmaceutical composition

The invention further provides a composition. In the preferred examples, the composition is a pharmaceutical composition comprising the antibody, or an active fragment, a fusion protein or an ADC thereof, or a corresponding immune cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated.

The formulated pharmaceutical composition may be administered by conventional routes, including (but not limited to): intratumoral, intraperitoneal, intravenous, or topical administration. Typically, the administration route of the pharmaceutical composition of the present invention is preferably injection or oral administration. The injection administration preferably includes intravenous injection, intramuscular injection, intraperitoneal injection, intradermal injection, or subcutaneous injection. The pharmaceutical composition is in one of a variety of conventional dosage forms in the art, preferably in solid, semi-solid or liquid form, and can be an aqueous solution, a non-aqueous solution or a suspension, and more preferably tablets, capsules, granules, injection or infusion, etc.

The antibody of the present invention can also be used for cell therapy by expressing the nucleotide sequence in a cell, for example, the antibody is used for chimeric antigen receptor T cell immunotherapy (CAR-T) and the like.

The pharmaceutical composition of the present invention is a pharmaceutical composition for prevention and/or treatment of diseases associated with abnormal TIM-3 expression or function.

The pharmaceutical composition according to the present invention can be directly used for binding to a TIM-3 protein molecule, and thus can be used for preventing and treating diseases such as tumors.

The pharmaceutical composition according to the present invention comprises a safe and effective amount (e.g. 0.001-99 wt %, preferably 0.01-90 wt %, more preferably 0.1-80 wt %) of the monoclonal antibody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 1 microgram per kilogram body weight to about 5 milligrams per kilogram body weight per day. Further, the polypeptide of the present invention can also be used in combination with the other therapeutic agents.

In the present invention, preferably, the pharmaceutical composition of the present invention further comprises one or more pharmaceutical carriers. The pharmaceutical carrier is a conventional pharmaceutical carrier in the art, and the pharmaceutical carrier can be any suitable physiologically or pharmaceutically acceptable pharmaceutical excipient. The pharmaceutical excipient is a conventional pharmaceutical excipient in the art, and preferably includes pharmaceutically acceptable excipients, fillers or diluents. More preferably, the pharmaceutical composition comprises 0.01-99.99% of the above-mentioned protein and 0.01-99.99% of the pharmaceutically acceptable carrier, wherein the percentage is the mass percentage of the pharmaceutical composition.

In the present invention, preferably, the administration amount of the pharmaceutical composition is an effective amount, and the effective amount is an amount that can alleviate or delay the progression of the disease, and the degenerative or traumatic condition. The effective amount can be determined on an individual basis and will be partly based on consideration of the symptoms to be treated and the results sought. Those skilled in the art can determine the effective amount by using the above-mentioned factors such as individual basis and using no more than conventional experiments.

When a pharmaceutical composition is used, a safe and effective amount of the immunoconjugate is administered to a mammal, wherein the safe and effective amount is usually at least about 10 micrograms per kilogram of body weight, and in most cases does not exceed about 50 mg/kg body weight, preferably the dose is about 10 micrograms/kg body weight to about 20 mg/kg body weight. Of course, the particular dose should also depend on various factors, such as the route of administration, patient healthy status, which are well within the skills of an experienced physician.

The present invention provides use of the above-mentioned pharmaceutical composition in the preparation of a medicine for preventing and/or treating diseases associated with abnormal TIM-3 expression or function. Preferably, the diseases associated with abnormal TIM-3 expression or function are cancers and autoimmune diseases.

### Method and composition for detecting TIM-3 protein in sample

The present invention also provides a method for detecting TIM-3 protein in a sample (for example, detecting cells over-expressing TIM-3), which comprises the following steps: contacting the above-mentioned antibody with a sample to be tested *in vitro,* and detecting whether the above-mentioned antibody binds to the sample to be tested, to form an antigen-antibody complex.

The meaning of overexpression is conventional in the art, which refers to the overexpression of RNA or protein of TIM-3 protein in the sample to be tested (due to increased transcription, post-transcriptional processing, translation, post-translational processing and protein degradation changes), and local overexpression and increased functional activity (such as in the case of increased enzymatic hydrolysis of the substrate) due to changes in protein transport mode (increased nuclear localization).

In the present invention, the detection method for detecting whether an antigen-antibody complex is formed is a conventional detection method in the art, preferably a flow cytometry (FACS) detection.

The present invention provides a composition for detecting TIM-3 protein in a sample, which comprises the above-mentioned antibody, recombinant protein, antibody conjugate, immune cell, or a combination thereof as an active ingredient. Preferably, it also comprises a compound composed of the functional fragments of the above-mentioned antibody as an active ingredient.

On the basis of conforming to common knowledge in the art, the above-mentioned preferred conditions can be combined arbitrarily to obtain preferred embodiments of the present invention.

### The main advantages of the invention are:

(1) The invention obtained fully human antibodies using rat-human chimeric antibody transgenic mice, and the obtained antibodies have a series of excellent characteristics:
   1) the variable region sequences are different from the existing antibody (homology < 92%);
   2) the obtained antibodies have strong affinity;
   3) the obtained antibodies have good activity of stimulating T cell activation;
(2) The transgenic mice used in the present invention can obtain fully human antibodies more easily than wild-type mice, therefore the immunogenicity of antibodies are reduced. And compared with transgenic mice of fully human antibodies, the number of antibodies obtained is larger, and the antibodies have strong affinity, good sequence diversity and high activity.
(3) Compared with antibodies obtained from phage library, the antibody of the present invention obtained by hybridoma technology has high affinity and good sequence expression.
(4) The invention has obtained antibodies with different sequences, which can specifically bind to TIM-3 with a binding activity lower than nanomolar. By reversing the inhibition of TIM-3 on the activation activity of T cells, T cells are activated to secrete IFN.

The invention is further illustrated by the following specific examples. It is to be understood that these examples are for illustrative purposes only and are not intended to limit the scope of the invention. The experimental methods without detailed conditions in the following examples are generally in accordance with the conditions described in the conventional conditions such as Sambrook. J et al. "Guide to Molecular Cloning Laboratory" (translated by Huang Peitang et al., Beijing: Science Press, 2002), or in accordance with the conditions recommended by the manufacturer (for example, product manuals). Percentages and parts are by weight unless otherwise stated. The experimental materials and reagents used in the following examples are commercially available unless otherwise specified.

The room temperature described in the examples is a conventional room temperature in the art, and is generally 10-30°C.

### EXAMPLE 1 Preparation of TIM-3 specific antibody

### (1) Preparation of immunogen

Immunogens including extracellular domain TIM-3 protein, TIM-3 recombinant cell line, expression plasmid of TIM-3 DNA vector and other immunogens are prepared.

Immunogen 1), the amino acid sequence 22-200 of the extracellular region of human TIM-3 protein (as shown in SEQ ID NO. 71 in the sequence listing) was cloned into the pCpC vector with human IgG Fc fragment (hFc) (purchased from Invitrogen, V044-50) and plasmids were prepared according to the established standard molecular biology methods. For specific methods, see Sambrook, J., Fritsch, E.F., and Maniatis T. (1989). Molecular Cloning: A Laboratory Manual, Second Edition (Plainview, New York: Cold Spring Harbor Laboratory Press). HEK293 cells (purchased from Invitrogen) were transiently transfected (PEI, Polysciences) and expanded using FreeStyle™ 293 (Invitrogen) at 37°C. After 4 days, the cell culture was collected, and the cell components were removed by centrifugation to obtain the culture supernatant containing the extracellular region of TIM-3 protein. The culture supernatant was loaded onto a protein A affinity chromatography column (Mabselect Sure, purchased from GE Healthcare), and an ultraviolet (UV) detector was used to monitor the change in ultraviolet absorbance (A280nm). After the sample was loaded, the protein affinity chromatography column was washed with PBS phosphate buffer (pH 7.2) until the UV absorption value returned to the baseline, and then eluted with 0.1M glycine hydrochloric acid (pH 2.5). The TIM-3 protein with hFc tag (CLTA-4-hFc) eluted from the protein affinity chromatography column was collected and dialyzed overnight with PBS phosphate buffer (pH 7.2) in a refrigerator at 4°C. The dialyzed protein was aseptically filtered by a 0.22 micron filter and stored at -80°C after subpackage to obtain purified immunogen human TIM-3-hFc protein. The immunogen TIM-3-hFc protein needed a series of quality control tests before use, such as tests of protein concentration, purity, molecular weight and biological activity.

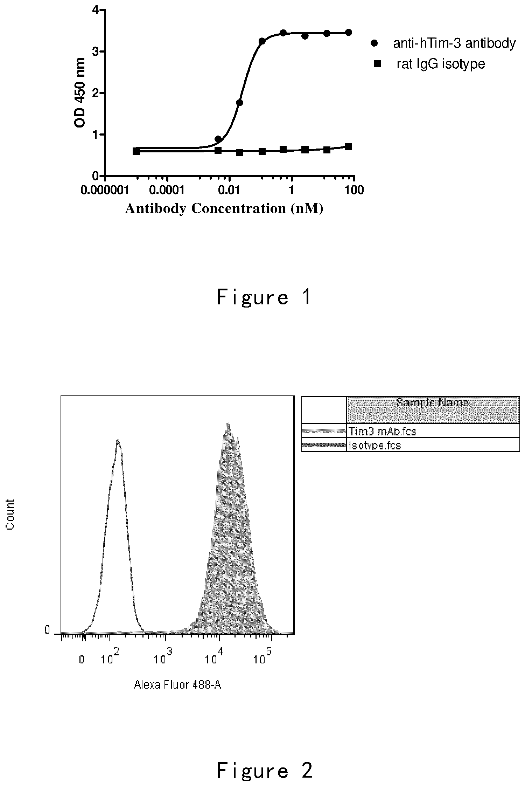

Among them, the binding activity of the immunogen TIM-3-hFc and the commercial anti-TIM-3 antibody is detected by ELISA, specifically:
The hFc-labeled TIM-3 protein (TIM-3-hFc, i.e., the immunogen) was diluted with PBS to 1µg/mL, and added 100µl/well to the ELISA microplate, and incubated overnight at 4°C. After blocked with ELISA blocking solution (containing 1% BSA, pH 7.4 PBS phosphate buffer solution, wherein the percentage is the mass percentage) at 37°C for two hours, the plated was added with gradient dilution of commercially available anti-TIM-3 antibody, and incubated at 37°C for 1 hour. The commercially available anti-TIM-3 antibody was purchased from the R&D system under trade number MAB2365. HRP (horseradish peroxidase) labeled secondary antibody was added, and after incubation at room temperature for 30 minutes, 100 microliters/well of TMB color development solution was added. After incubated at room temperature for 15 minutes, the plate was added with 50 microliters of IN hydrochloric acid to stop the color reaction, and read with an ELISA plate reader for the OD450nm reading. The plate needed to be washed after each step. Wherein the negative control antibody was rat IgG, and the results are shown in Figure 1 and Table 1.

**Table 1 Binding activity of TIM-3-hFc protein to its commercially available antibody**

| Coating antigen | Antibody concentration (nM) | Commercially available anti- TIM-3 antibody | | Rat IgG | |
|---|---|---|---|---|---|
| hTIM-3-hFC 1µg/ml | 66.7 | 3.48 | 3.44 | 0.72 | 0.70 |
| | 13.34 | 3.45 | 3.42 | 0.63 | 0.63 |
| | 2.67 | 3.32 | 3.42 | 0.69 | 0.58 |
| | 0.53 | 3.52 | 3.38 | 0.63 | 0.65 |
| | 0.11 | 3.23 | 3.27 | 0.60 | 0.59 |
| | 0.021 | 1.78 | 1.75 | 0.55 | 0.58 |
| | 0.0043 | 0.92 | 0.86 | 0.63 | 0.59 |
| | 0 | 0.60 | 0.59 | 0.61 | 0.59 |

The results shows that the binding of TIM-3 to commercially available antibody against TIM-3 protein at protein level changed with the concentration of antibody.

Immunogen 2), the human TIM-3 full-length amino acid sequence was cloned into pIRES vector (purchased from Clontech) and the plasmid was prepared. After plasmid transfection on HEK293 cell line and CHOK1 cell line (both purchased from Invitrogen) (transfection was preformed using X-treme GENE HP DNA Transfection Reagent, which was purchased from Roche, Cat #06 366 236 001, and operated according to the instructions) , cells were selectively cultured in DMEM medium containing 10% (w/w) FBS and containing 0.5 µg/ml puromycin for 2 weeks. Subcloning was conduted in 96-well culture plate by a limiting dilution method, and the plate was placed at 37 °C, 5% (v/v) CO₂. After about 2 weeks, some of the monoclonal wells were selected and amplified into 6-well plates. The amplified clones were stained with known TIM-3 antibodies and screened by flow cytometry. The monoclonal cell line with better growth and higher fluorescence intensity were continued to be expanded in culture and cryopreserved in liquid nitrogen, to obtain the immunogen TIM-3 recombinant cell line. The specific selection results are shown in Table 2 and Figure 2. In Table 2, positive cells (%) refer to the percentage of positive cells number in the total cells number, and MFI is the average fluorescence intensity value of the measured cell population. Figure 2 shows that HEK293 cells have a high level of TIM-3 expression.

**Table 2 The FACS screening detection results of HEK293 cells transfected with TIM-3 gene**

| Number | Recombinant cell clone ID | IgG control | | Anti-TIM-3 mAb | |
|---|---|---|---|---|---|
| | | Gated (%) | MFI | Gated (%) | MFI |
| 1 | 1B6 | 0.88 | 26 | 95.46 | 11684 |
| 2 | 1A5 | 0.79 | 22 | 94.57 | 12657 |
| 3 | 3C3 | 0.89 | 22 | 98.02 | 15499 |
| 4 | 3D2 | 0.95 | 22 | 99.01 | 13486 |
| 5 | 4C5 | 0.85 | 24 | 99.42 | 11235 |

Immunogen 3), TIM-3 full-length amino acid sequence cDNA was cloned into a pCDNA3.1 vector and coated on a 1.0um gold colloidal bullet, and immunized with Helios gene gun (Bio-rad). The detailed method was developed according to the instructions of Helios gene gun.

### (II) Preparation of hybridoma cells and screening of antibody

Harbour transgenic mice are introduced with human immunoglobulin variable region genes and rat immunoglobulin constant region genes, while the Ig expression of the mice own is silenced (F.G. Franklin, et al, patent #WO 2010/070263 A1). The transgenic mice can produce immune response and antibody titer equivalent to that produced by normal mice (such as Balb/c) after being immunized with antigen.

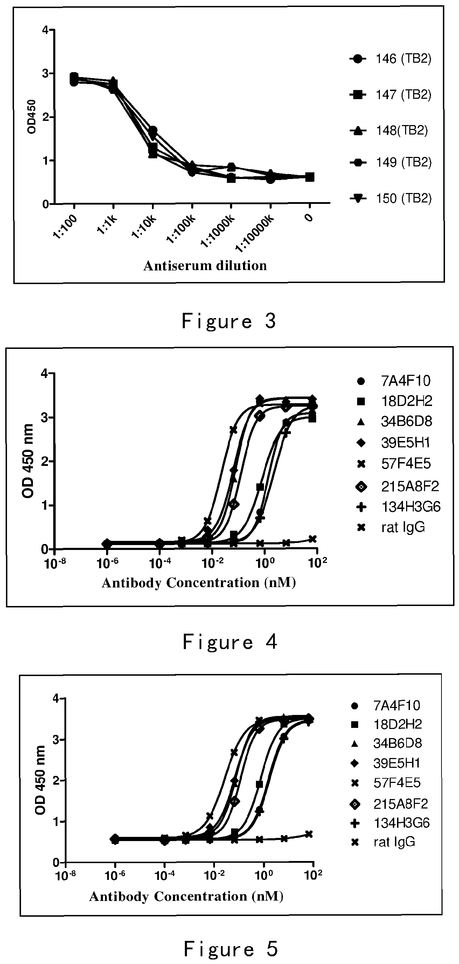

A. 6-8 weeks old Harbour human antibody transgenic mice (purchased from Beijing Weitong Lihua Company) were used for immunization by immunogen 1), and the mice were raised under SPF conditions. At the initial immunization, the immunogen TIM-3-hFc protein was emulsified with Freund's complete adjuvant and then injected intraperitoneally with 0.25 ml, that is, 100 micrograms of immunogen protein was injected into each mouse. During the booster immunization, immunogen protein was emulsified with Freund's incomplete adjuvant and then injected intraperitoneally with 0.25 ml, that is, 50 micrograms of immunogen protein was injected into each mouse. The interval between the initial immunization and the first boosting immunization was 2 weeks. After that, the intervals between each boosting immunization were 3 weeks. Blood was collected 1 week after each boosting immunization, and the antibody titer and specificity of immunogen protein in the serum were detected by ELISA and FACS. The results are shown in Figure 3 and Table 3.

**Table 3 Detection of serum antibody titer in mice after TIM-3-hFC protein immunization by ELISA**

| OD₄₅₀ₙₘ | Serum dilution | | | | | | |
|---|---|---|---|---|---|---|---|
| Batch | 1:100 | 1:10³ | 1:10⁴ | 1:10⁵ | 1:10⁶ | 1:10⁷ | Blank control |
| 146 (TB2) | 2.79 | 2.75 | 1.69 | 0.84 | 0.6 | 0.55 | 0.6 |
| 147 (TB2) | 2.86 | 2.75 | 1.3 | 0.8 | 0.58 | 0.61 | 0.6 |
| 148 (TB2) | 2.9 | 2.82 | 1.14 | 0.88 | 0.83 | 0.69 | 0.59 |
| 149 (TB2) | 2.93 | 2.61 | 1.18 | 0.71 | 0.85 | 0.64 | 0.61 |
| 150 (TB2) | 2.9 | 2.66 | 1.54 | 0.71 | 0.58 | 0.59 | 0.6 |

Table 3 shows that the serum of mice immunized with TIM-3-hFc had different degrees of binding to the immunogen, showing antigen-antibody reaction, with the highest dilution being about 10,000. Wherein, the blank control was 1% (w/w) BSA, and the batch referred to the mice serum on the seventh day after the second boosting immunization. The data in the table is the value of OD₄₅₀ₙₘ.

B. 6-8 weeks old Harbour human antibody transgenic mice (purchased from Beijing Weitong Lihua Company) were used for immunization by immunogen 2), and the mice were raised under SPF conditions. The obtained HEK293-h TIM-3 stable cell line containing human TIM-3 was expanded to a confluence of 90% in a T-75 cell culture flask, and the medium was aspirated. Cells were washed with DMEM basal medium (purchased from Invitrogen) twice, and then treated with enzyme-free cell dissociation solution (purchased from Invitrogen) at 37°C until the cells were detached from the wall of the culture dish, and then the cells were collected. Cells were washed twice with DMEM basal medium and counted, and then diluted with phosphate buffer (pH 7.2) to 2×10⁷ cells per ml. Each mouse was intraperitoneally injected with 0.5 ml of cell suspension during each immunization. The interval between the first and the second immunization was 2 weeks. After that, the intervals between each subsequent immunization were 3 weeks. Except for the initial immunization, blood was collected one week after each immunization, and the antibody titer and specificity in the serum were detected by ELISA.

C. 6-8 weeks old Harbour human antibody transgenic mice (purchased from Beijing Weitong Lihua Company) were used for immunization by immunogen 3), and the mice were raised under SPF conditions. All mice were immunized with the gene gun through the abdomen for 3-4 times, 3-4 shots each time, 1.0 µg cDNA amount per shot. The interval between the first immunization and the first booster immunization was 2 weeks. After that, the intervals between each subsequent immunization were 3 weeks. Blood was collected 7 days after each boost, and the antibody titer in the serum was detected by ELISA.

Usually, the ELISA titer of most mice can reach more than 1: 1000 after 2-3 times of immunizations. Table 3 and Figure 3 show the results of the antibody titer in serum detected by ELISA after immunization with TIM-3-hFC protein.

The results show that: after 3 times of immunization with the immunogen, most mice had an ELISA titer of more than 1:100, indicating that mice had a better humoral immune response to the immunogen, and their spleen cells can be used for Hybridoma cell preparation.

Mice whose titers meet the requirements can be selected for cell fusion and hybridoma preparation. Before cell fusion, each mouse was injected intraperitoneally with 50-100 micrograms of purified TIM-3-hFc, for the last immunization. After 3-5 days, the mice were sacrificed and splenocytes were collected. Cells were washed 3 times by centrifugation at 1000 revolutions per minute in DMEM basal medium, and then mixed with mouse myeloma cells SP2/0 (purchased from ATCC) at a ratio of 5:1 according to the number of viable cells. High-efficiency electrofusion or PEG method (see METHODS IN ENZYMOLOGY, VOL. 220) was used for cell fusion. The fused cells were diluted into DMEM medium containing 20% fetal bovine serum and 1×HAT, wherein the percentage was the mass percentage. Then the cell solution was added as 1× 10⁵/200 microliters per well to a 96-well cell culture plate, and put in a 5% CO₂, 37°C incubator, wherein the percentage was the volume percentage. After 14 days, ELISA and Acumen (microwell plate cell detection method) were used to screen the supernatants in cell fusion plate. The positive clones with OD₄₅₀ₙₘ>1.0 in ELISA and MFI value>100 in Acumen were expanded to a 24-well plate, in the medium of DMEM (Invitrogen) containing 10% (w/w) of fetal bovine serum, and cultured at 37°C and 5% (v/v) CO₂. After 3 days of culture, the culture solution expanded in the 24-well plate was centrifuged. The supernatant was collected, and the supernatant was analyzed for antibody subtypes. The binding activity of the antibody to TIM-3 protein and TIM-3 positive cells was determined by ELISA and FACS.

According to the results of 24-well plate screening, hybridoma cells with OD₄₅₀ₙₘ>1.0 in the ELISA experiment and MFI value>50 in the FACS experiment were selected as qualified positive clones. The qualified hybridoma cells were selected to subclone in a 96-well plate by limiting dilution method, and cultured in a DMEM medium (purchased from Invitrogen) containing 10% (w/w) FBS, at 37°C, 5% (v/v) CO₂. 10 days after subcloning, ELISA and Acumen were used for preliminary screening, and positive monoclones were selected and amplified to a 24-well plate to continue culture. After 3 days, the positive antigen binding was determined by FACS to evaluate the biological activity (the evaluation standard was OD₄₅₀ₙₘ>1.0 in ELISA experiment, MFI value > 100 in FACS experiment).

According to the test results of the 24-well plate samples, the positive clones were expanded in DMEM (purchased from Invitrogen) medium containing 10% (w/w) FBS at 37°C and 5% (v/v) CO₂. The cells were suspended in freezing solution [DMEM containing 20% (w/w) FBS and 10% (w/w) DMSO], and cryopreserved in liquid nitrogen according to conventional methods, to obtain hybridoma cells of the present invention, which can be used for subsequent antibody production, purification and amino acid sequence determination.

### EXAMPLE 2 Identification of chimeric antibody

### (I) Enzyme-linked immunosorbent assay (ELISA) detection of the binding of antibodies to TIM-3 protein

The amino acid sequence 22-200 of the extracellular domain of the human TIM-3 protein described in the step of Example 1 (as shown in SEQ ID NO. 71 in the sequence listing) was cloned into a pCpC vector having a human IgG Fc fragment (hFc), and transfected into HEK293 cells. The cell culture medium was collected, and purified to obtain the human TIM-3 protein with hFc tag (herein referred to as hTIM-3-hFc protein). The amino acid sequence 22-201 of the extracellular domain of the monkey TIM-3 protein (as shown in SEQ ID NO. 72 in the sequence listing) was cloned into a pCpC vector having a human IgG Fc fragment (hFc), and transfected into HEK293 cells. The cell culture medium was collected, and purified to obtain the monkey TIM-3 protein with hFc tag (herein referred to as cTIM-3-hFc protein). The amino acid sequence 22-191 of the extracellular domain of murine TIM-3 protein (as shown in SEQ ID NO. 73 in the sequence listing) was cloned into a pCpC vector having a human IgG Fc fragment (hFc), and transfected into HEK293 cells. The cell culture medium was collected, and purified to obtain the murine TIM-3 protein with hFc tag (herein referred to as mTIM-3-hFc protein). Purified human, monkey, and mouse TIM-3 extracellular domain proteins (hTIM-3-hFc, cTIM-3-hFc, and mTIM-3-hFc) were diluted with PBS to a final concentration of 1.0 µg/ml and then added to a 96-well ELISA plate with 100 µl per well. The plate was sealed with plastic film and incubated overnight at 4 °C. The plate was washed 4 times with the plate washing solution (PBS + 0.01% Tween20) on the next day, and added with the blocking solution (PBS + 0.01% Tween20 + 1% BSA) and sealed at room temperature for 1-2 hours. The blocking solution was poured out. 50-100 µl of the antibody sample to be tested was added to each well. The plate was incubated at 37 °C for 1-2 hours, and washed 2-3 times with plate washing solution (PBS+0. 01% Tween20). HRP (horseradish peroxidase) labeled secondary antibody was added. The plate was incubated at 37 °C for 1-2 hours, and washed 2-3 times with plate washing solution (PBS+0. 01% Tween20). 100µl of TMB substrate was added to each well. After incubated at room temperature for 15-30 minutes, the plate was added with 100µl of stop solution (1.0N HCl) to each well. An ELISA plate reader (TiterMax 384plus, Molecular Device) was used to read the A450nm value. The results are shown in Figures 4, 5 and 6, Tables 4, 5 and 6, the antibody to be tested can bind to human and monkey TIM-3 extracellular domain proteins to varying degrees, but cannot bind to mouse TIM-3 proteins. Wherein, the IgG control is rat IgG, and the data in the table are A450nm values.

**Table 4 Activity of TIM-3 antibodies reacting with human TIM-3 extracellular domain protein in the enzyme-linked immunosorbent assay.**

| Clone ID | OD450 | | | | | | | |
|---|---|---|---|---|---|---|---|---|
| | Antibody concentration (nM) | | | | | | | |
| | 66.667 | 6.6667 | 0.6667 | 0.0667 | 0.0067 | 0.0007 | 0.0001 | 0 |
| 7A4F10 | 3.08 | 2.92 | 0.86 | 0.21 | 0.13 | 0.12 | 0.12 | 0.11 |
| 18D2H2 | 2.93 | 2.86 | 1.33 | 0.35 | 0.16 | 0.12 | 0.12 | 0.11 |
| 34B6D8 | 3.42 | 3.40 | 3.41 | 1.62 | 0.33 | 0.15 | 0.12 | 0.12 |
| 39E5H1 | 3.39 | 3.34 | 3.41 | 1.78 | 0.42 | 0.16 | 0.13 | 0.12 |
| 57F4E5 | 3.26 | 3.29 | 3.31 | 2.83 | 0.63 | 0.19 | 0.13 | 0.12 |
| 134H3G6 | 3.15 | 2.57 | 0.69 | 0.20 | 0.13 | 0.13 | 0.12 | 0.12 |
| 215A8F2 | 3.17 | 3.15 | 3.06 | 1.00 | 0.28 | 0.14 | 0.12 | 0.11 |
| Mouse IgG control | 0.22 | 0.13 | 0.13 | 0.12 | 0.12 | 0.12 | 0.11 | 0.11 |

**Table 5 Activity of TIM-3 antibodies reacting with monkey TIM-3 extracellular domain protein in the enzyme-linked immunosorbent assay.**

| Clone ID | **OD450** | | | | | | | |
|---|---|---|---|---|---|---|---|---|
| | Antibody concentration (nM) | | | | | | | |
| | 66.667 | 6.6667 | 0.6667 | 0.0667 | 0.0067 | 0.0007 | 0.0001 | 0 |
| 7A4F10 | 3.46 | 3.12 | 1.35 | 0.70 | 0.57 | 0.55 | 0.54 | 0.54 |
| 18D2H2 | 3.44 | 3.34 | 1.89 | 0.74 | 0.62 | 0.54 | 0.53 | 0.55 |
| 34B6D8 | 3.52 | 3.55 | 3.44 | 1.92 | 0.78 | 0.61 | 0.57 | 0.58 |
| 39E5H1 | 3.47 | 3.50 | 3.41 | 1.98 | 0.85 | 0.61 | 0.57 | 0.59 |
| 57F4E5 | 3.47 | 3.48 | 3.45 | 2.68 | 1.13 | 0.64 | 0.58 | 0.56 |
| 134H3G6 | 3.43 | 3.02 | 1.22 | 0.64 | 0.56 | 0.55 | 0.54 | 0.55 |
| 215A8F2 | 3.49 | 3.45 | 3.26 | 1.50 | 0.70 | 0.55 | 0.52 | 0.57 |
| Mouse IgG control | 0.68 | 0.58 | 0.55 | 0.54 | 0.57 | 0.55 | 0.54 | 0.55 |

**Table 6 Activity of TIM-3 antibodies reacting with mouse TIM-3 extracellular domain protein in the enzyme-linked immunosorbent assay.**

| Clone ID | **OD450** | | | | | | | |
|---|---|---|---|---|---|---|---|---|
| | Antibody concentration (nM) | | | | | | | |
| | 66.667 | 6.6667 | 0.6667 | 0.0667 | 0.0067 | 0.0007 | 0.0001 | 0 |
| 7A4F10 | 0.66 | 0.67 | 0.67 | 0.62 | 0.64 | 0.64 | 0.65 | 0.63 |
| 18D2H2 | 0.85 | 0.69 | 0.66 | 0.67 | 0.63 | 0.64 | 0.64 | 0.62 |
| 34B6D8 | 1.10 | 0.74 | 0.65 | 0.68 | 0.65 | 0.65 | 0.62 | 0.66 |
| 39E5H1 | 0.90 | 0.68 | 0.66 | 0.68 | 0.64 | 0.63 | 0.64 | 0.62 |
| 57F4E5 | 0.74 | 0.69 | 0.64 | 0.64 | 0.68 | 0.67 | 0.63 | 0.64 |
| 134H3G6 | 0.70 | 0.67 | 0.66 | 0.65 | 0.64 | 0.62 | 0.64 | 0.64 |
| 215A8F2 | 0.77 | 0.68 | 0.65 | 0.67 | 0.64 | 0.67 | 0.63 | 0.63 |
| Mouse IgG control | 0.75 | 0.65 | 0.66 | 0.64 | 0.62 | 0.67 | 0.63 | 0.60 |

### (II) Detection of the binding of antibodies to TIM-3 expressing cells by Flow cytometry (FACS)

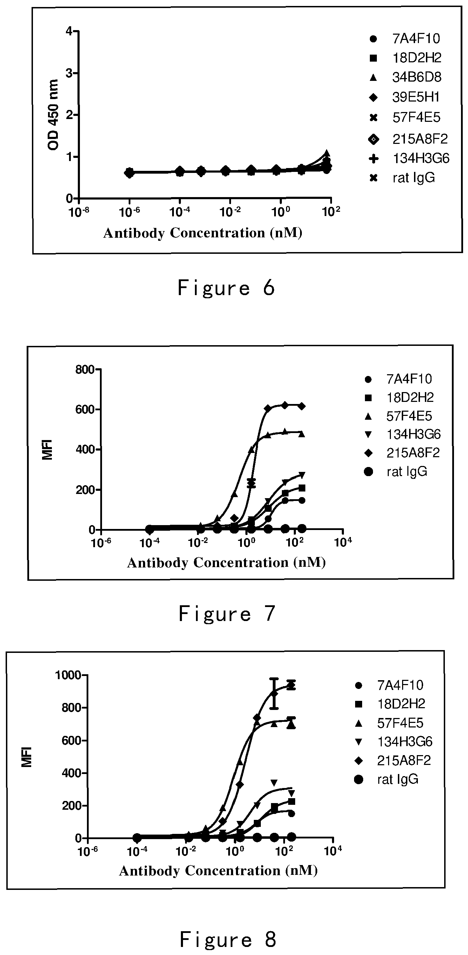

The pIRES plasmid containing the full-length nucleotide sequence encoding human TIM-3 as described in Example 1 was transfected into CHOK1 cell line to obtain a CHOK1 stable cell line containing human TIM-3 (herein referred to as CHOK1-hTIM-3 stable cell line). The pIRES plasmid containing the full-length gene of monkey TIM-3 was transfected into CHOK1 cell line to construct a CHOK1 stable cell line containing monkey TIM-3 (herein referred to as CHOK1-cTIM-3 stable cell line). The CHOK1-hTIM-3 stable cell line and CHOK1-cTIM-3 stable cell line were expanded to a confluence of 90% in a T-75 cell culture flask. The medium was aspirated, and the cells were washed with HBSS (Hanks' Balanced Salt Solution) 1-2 times, and then treated with an enzyme-free cell dissociation solution (Versene solution: Life technology) and the cells were collected. The cells were washed with HBSS buffer for 1-2 times. After counted, the cells were diluted with HBSS to 1-2×10⁶ cells per ml, added with 1% goat serum blocking solution, incubated on ice for 20-30 minutes, and then washed with HBSS by centrifugation 2 times. The collected cells were suspended in the FACS buffer (HBSS+1%BSA) to 2×10⁶ cells/ml, added as 100 microliters per well to a 96-well FACS reaction plate, added with 100 microliters per well of the antibody sample to be tested, incubated on ice for 1-2 hours. The plate was washed twice with the FACS buffer by centrifugation, added with 100 microliters of fluorescent (Alexa 488)-labeled secondary antibodies per well, and incubated on ice for 0.5-1.0 hours. The plate was washed 2-3 times with FACS buffer by centrifugation, added with 100µl fixative solution (4% Paraformaldehyde) per well to suspend the cells. 5-10 minutes later, it was washed 1-2 times with FACS buffer by centrifugation. The cells were suspended with 100 microliters of FACS buffer, FACS (FACSCalibur, BD) was used for detection and the results were analyzed. The results are shown in Figures 7 and 8, Tables 7 and 8, wherein the IgG control is rat IgG, and the data in the table are the average fluorescence intensity values of the cell populations measured by MFI. The results show that the antibody to be tested can bind to human or monkey TIM-3 protein on the cell surface.

**Table 7 Binding reaction of TIM-3 antibody to CHOK1-hTIM-3 detected by FACS**

| Clone ID | Mean fluorescence intensity | | | | | | | |
|---|---|---|---|---|---|---|---|---|
| | Antibody concentration (nM) | | | | | | | |
| | 200 | 40 | 8 | 1.6 | 0.32 | 0.064 | 0.013 | 0 |
| 7A4F10 | 145.2 | 144.2 | 56.2 | 15.4 | 6.8 | 4.3 | 3.3 | 2.9 |
| 18D2H2 | 211.9 | 185.4 | 106.9 | 40.2 | 15.6 | 6.6 | 4.2 | 2.9 |
| 57F4E5 | 484.9 | 484.6 | 478.4 | 389.5 | 176.7 | 58.1 | 21.2 | 2.9 |
| 134H3G6 | 261.3 | 229.6 | 139.0 | 50.0 | 17.7 | 8.2 | 4.7 | 3.0 |
| 215A8F2 | 612.6 | 610.3 | 607.5 | 213.5 | 55.6 | 18.7 | 8.6 | 3.0 |
| rIgG control | 3.8 | 3.3 | 3.1 | 3.0 | 3.0 | 3.0 | 3.0 | 3.0 |

**Table 8 Binding reaction of TIM-3 antibody to CHOK1-cTIM-3 detected by FACS**

| Clone ID | Mean fluorescence intensity | | | | | | | |
|---|---|---|---|---|---|---|---|---|
| | Antibody concentration (nM) | | | | | | | |
| | 200 | 40 | 8 | 1.6 | 0.32 | 0.064 | 0.013 | 0 |
| 7A4F10 | 146.7 | 163.3 | 90.0 | 29.1 | 11.3 | 5.9 | 4.1 | 3.2 |
| 18D2H2 | 203.2 | 186.2 | 92.2 | 35.8 | 14.4 | 6.9 | 4.3 | 3.2 |
| 57F4E5 | 675.2 | 698.6 | 704.0 | 460.5 | 184.8 | 59.6 | 22.2 | 3.2 |
| 134H3G6 | 283.4 | 332.1 | 194.9 | 83.5 | 28.8 | 11.6 | 6.2 | 3.2 |
| 215A8F2 | 961.1 | 975.6 | 732.6 | 330.3 | 107.5 | 34.7 | 13.6 | 3.2 |
| rIgG control | 4.8 | 3.7 | 3.8 | 3.6 | 3.5 | 3.4 | 3.3 | 3.2 |

### (III) Detection of the effect of TIM-3 antibody on lymphocyte activity by lymphocyte stimulation test

In the lymphocyte stimulation test, TIM-3 antibodies block the binding of TIM-3 protein and its receptor to relieve the inhibition of T lymphocyte activity, thereby stimulating the proliferation of T cells.

### 1. Isolation of Peripheral blood monocytes lymphocyte PBMCs from the whole blood using Ficoll

The freshly obtained whole blood was diluted with phosphate buffer PBS at a volume ratio of 1:1, to obtain diluted whole blood. A sterile pipette was used to gently spread the diluted whole blood on the surface of Ficoll (purchased from GE Healthcare). The volume ratio of Ficoll to diluted whole blood was 3:4. The solution was mixed without shaking, gradient centrifuged at 400g at room temperature 20 °C for 30 minutes. The solution in the centrifuge tube after centrifugation was divided into three layers, wherein the upper layer was plasma and the middle layer was milky white, which was mononuclear lymphocytes. A sterile pipette was used to gently aspirate the middle layer cells, collected in a new centrifuge tube, diluted to three times of volume with PBS phosphate buffer, and centrifuged at 100g at room temperature for 10 minutes, then the suprenatant was discarded. The lymphocytes were resuspended to 10mL in PBS phosphate buffer, and the previous steps were repeated to remove the platelets. Finally, the lymphocytes were resuspended in 10 mL of multi-component RPMI1640 medium (purchased from Invitrogen) containing 10% fetal bovine serum for use, namely peripheral blood mononuclear lymphocytes PBMCs, and the percentages were mass percentages.

### 2. Pre-stimulation of PBMC cells mediated by OKT3

Commercial OKT3 antibody (eBioscience Cat # 16-0037-81) was diluted with PBS to a final concentration of 1.0 µg/ml and then added to a 6-well cell culture plate at 2ml per well. The plate was sealed with plastic film, incubated overnight at 4 °C, and washed with PBS three times the next day. The isolated PBMC cells were inoculated into the 6-well plate, and cultured in an incubator at 37 °C, 5% CO₂ for 72 hours.

### 3. CHOK1-OS8 cells treated with mitomycin

The single chain variable fragment (scFv) of anti-human CD3 monoclonal antibody OKT3 was fused to the C-terminal domain (113-220) of mouse CD8a to construct a T cell conjugate, i.e. a membrane dislocation chimeric antibody (OS8). The C-terminal domain of the mouse CD8a includes a hinge, a transmembrane and cytoplasmic domain, so that the anti-CD3 scFv fragment can be dislocated to the cell surface as a T cell activator. The plasmid expressing the recombinant fusion protein OS8 was transfected into a CHOK1 cell line to obtain a CHOK1 stable cell line expressing OS8 (a T cell activating molecule) on the cell surface (hereinafter referred to as a CHOK1-OS8 stable cell line). Before use, CHOK1-OS8 cells were treated with 10 µg/mL mitomycin at 37 °C for 2 hours, and washed three times with PBS to remove residual mitomycin.

### 2. Stimulation test of PBMC mediated by CHOK1-OS8

Before the test, the TIM-3 antibody to be tested diluted in equal volume ratio was prepared to obtain a sample solution to be tested.

The pre-stimulated peripheral blood mononuclear lymphocytes PBMCs were plated with 1×10⁵ cells, 100 microliters per well, on a 96-well cell culture plate, and then the test sample solution was added to the culture plate and incubated at room temperature for 30 minutes. Finally, CHOK1-OS8 cells were added at 2.5 × 10⁴ cells of 50µl per well to a 96-well cell culture plate, ensuring a volume of 200 µl per reaction well. The reaction plate was placed in a 37°C, 5% CO₂ incubator. After 20 hours of culture, the supernatant was collected to obtain cell supernatant, and cryopreserved at -20 °C. The percentage was volume percentage.

### 5. Detection of cytokine interleukin IFN in cell supernatant

The enzyme-linked immunosorbent assay (ELISA) of the cytokine interferon IFN-γ in cell supernatant was preformed using the R&D system related detection kit human IFN-γ DuoSet ELISA (DY285), and operated in accordance with the instructions. All test reagents except the tested antibodies were provided by the test kit.

The detection of enzyme-linked immunosorbent assay (ELISA) of the content of cytokine IFN-γ in cell supernatant was preformed using double antibody sandwich ELISA kit (purchased from R&D Systems, IFN-γ Cat # DY285). The experimental operation was strictly in accordance with the requirements of the kit instructions, and all test reagents were provided by the kit. The specific experiment was briefly described as follows. The IFN-γ polyclonal antibody was coated on the ELISA microwell plate, sealed with plastic film and incubated overnight at 4 °C. The plate was washed 4 times with the plate washing solution on the next day, and added with the blocking solution and blocked at room temperature for 1-2 hours. The plate was washed 4 times with the plate washing solution. The cell supernatant obtained in step 4 was used as the test sample. The standard and the test sample were incubated at room temperature for 2 hours. 400 microliters of washing solution was added to each well, the plate washing was repeated 4 times. Then horseradish peroxidase-labeled antibody against human IFN-γ was added, and incubated for 2 hours at room temperature to form an immune complex with the IFN-γ on the microplate and the microwells were washed. The substrate was added for color development, protected from light at room temperature for 30 minutes. Finally the stop solution was added, and the absorbance at A450nm was measured with a microplate reader.

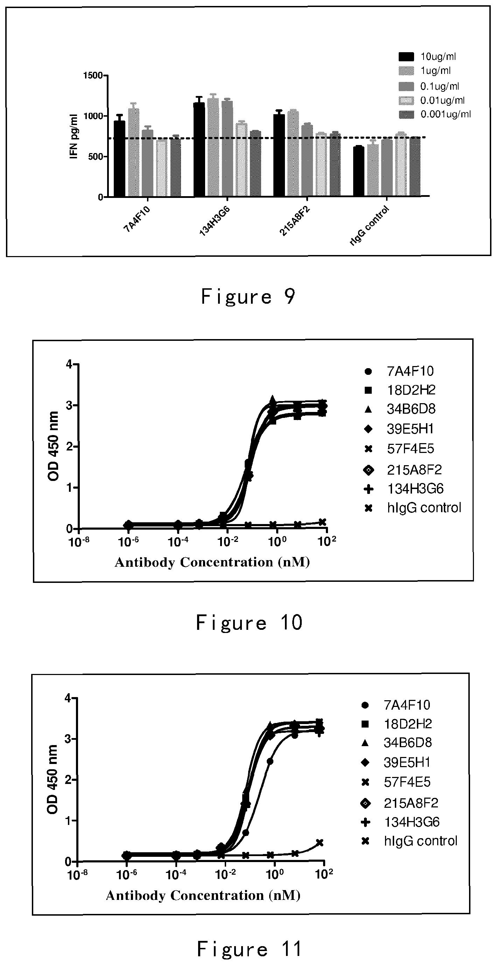

The effect of TIM-3 antibody on IFN-γ secretion in the PBMC stimulation experiment was detected. The results are shown in Figure 9 and Table 9. The IgG control is rat IgG, and the data in the table is IFN-γ value (pg/mL).

**Table 9 Effect of TIM-3 antibody on IFN-γ secretion in OKT3-dependent PBMC activation experiment**

| Clone ID | IFN production, pg/ml | | | | | | | | | |
|---|---|---|---|---|---|---|---|---|---|---|
| | 10µg/ml | | 1µg/ml | | 0.1µg/ml | | 0.001µg/ml | | 0.001µg/ml | |
| | N=1 | N=2 | N=1 | N=2 | N=1 | N=2 | N=1 | N=2 | N=1 | N=2 |
| 7A4F10 | 847.5 | 1010.7 | 1010.4 | 1157.0 | 757.7 | 873.4 | 672.1 | 718.6 | 666.3 | 756.4 |
| 134H3G6 | 1073.2 | 1231.3 | 1266.0 | 1132.9 | 1204.6 | 1133.9 | 862.3 | 932.3 | 809.7 | 792.8 |
| 215A8F2 | 943.0 | 1065.3 | 1019.3 | 1072.1 | 840.1 | 901.0 | 781.6 | 752.7 | 790.8 | 740.9 |
| rIgG control | 619.6 | 582.5 | 693.5 | 573.8 | 717.5 | 663.7 | 737.7 | 786.3 | 727.4 | 714.3 |

The results show that the antibodies to be tested can enhance IFN-γ secretion of PBMC in the PBMC lymphocyte stimulation experiment.

### Example 3 Determination of amino acid sequences of light and heavy chain variable regions

Isolation of total RNA: After the supernatant obtained from the subclonal culture of Example 1 was tested for antigen binding (that is, after the verification and activity determination in Example 2), 5×10⁷ hybridoma cells were collected by centrifugation, added with 1mL Trizol and mixed well and transferred to a 1.5mL centrifuge tube, and allowed to stand at room temperature for 5 minutes. The tube was added with 0.2mL chloroform, shaked for 15 seconds, let stand for 2 minutes, and centrifuged at 12000g at 4°C for 5 minutes. The supernatant was taken and transferred to a new 1.5mL centrifuge tube. 0.5 mL of isopropanol was added, and the liquid in the tube was gently mixed, and let stand at room temperature for 10 minutes. After centrifuge at 12000g for 15 minutes at 4°C, the supernatant was discarded. 1 mL of 75% ethanol (the percentage was volume percentage) was added, and the precipitate was gently washed, centrifuged at 12000g at 4°C for 5 minutes. The supernatant was discarded, and the precipitate was dried, and added with DEPC-treated H₂O for dissolution (55°C water bath to promote dissolution for 10 minutes). The total RNA was obtained.

Reverse transcription and PCR: 1µg of total RNA was taken, and a 20ul system was configured, added with reverse transcriptase and reacted at 42°C for 60 minutes, and the reaction was terminated at 7° C for 10 minutes. 50µl PCR system was configured, comprising 1µl cDNA, 25pmol of each primer, 1µl DNA polymerase and a matching buffer system, 250µmol dNTPs. PCR program was set, comprising pre-denaturation 95° C 3min, denaturation 95° C 30s, annealing 55° C 30s, extension 72° C 35s, and extension at 72° C for 5 min after 35 cycles. And the PCR product was obtained. The kit used for reverse transcription was PrimeScript RT Master Mix, purchased from Takara, and the catalog number was RR036; the kit used for PCR was Q5 ultra-fidelity enzyme, purchased from NEB, and the catalog number was M0492.

Cloning and sequencing: 5µl of PCR product was taken for agarose gel electrophoresis detection, and the column recovery kit was used to purify the positive samples. Wherein, the recovery kit was NucleoSpin® Gel & PCR Clean-up, purchased from MACHEREY-NAGEL, and the catalog number was 740609. Ligation reaction was performed in a 10µl of reaction system containing sample 50ng, T vector 50ng, ligase 0.5µl, and buffer 1µl and reacted for half an hour at 16°C to obtain the ligation product. Wherein, the ligation kit was T4 DNA ligase, purchased from NEB, and the catalog number was M0402. 5µl of ligation product was taken and added into 100µl of competent cells (Ecos 101competent cells, purchased from Yeastern, catalog number FYE607) in ice bath for 5 minutes. Then heat shock was carrited out in a 42°C water bath for 1 minute, and put back on ice for 1 minute, added with 650µl of antibiotic-free SOC medium, resuscitated on a 37°C shaker at 200RPM for 30 minutes. 200µl of the culture was taken and spreaded on LB solid medium containing antibiotics and incubated overnight at 37°C in an incubator. The next day, the primers M13F and M13R on the T vector were used to configure a 30µl PCR system to perform colony PCR. A pipette tip was used to dip the colony into the PCR reaction system and pipette, and 0.5µl was aspirated onto another piece of 100nM ampicillin LB solid petri dish to save the strain. After the PCR reaction, 5µl was taken out for agarose gel electrophoresis detection, and the positive samples were sequenced. Wherein, the steps of sequencing can be found in Kabat, Sequences of Proteins of Immunological Interest, National Institutes of Health, Bethesda, Md. (1991).

The sequencing results are shown in the sequence information of the present invention in the appendix, and the sequence information of the heavy chain CDR1-3 and light chain CDR1-3 of 7 clones is shown in Table 18.

### EXAMPLE 4 Transformation and preparation of fully human antibody IgG

(I) Plasmid construction and preparation: purified TIM-3 antibody from the culture supernatant of hybridoma cells had been obtained in Example 2, and according to the sequencing results of Example 3, the sequences of heavy chain variable region and light chain variable region of TIM-3 antibody was clear. The heavy chain variable region sequence of the TIM-3 antibody was recombined into an expression vector containing the signal peptide and the human heavy chain antibody IgG4 constant region (the expression vector was purchased from Invitrogen), and the light chain variable region sequence of the TIM-3 antibody was recombined into an expression vector containing the signal peptide and the human antibody light chain kappa constant region. The recombinant plasmid was obtained and verified by sequencing (the sequencing method was the same as that in Example 3). Plasmids with a mass of more than 500µg were extracted using alkaline lysis kit (purchased from MACHEREY-NAGEL) to increase the purity, filtered through a 0.22µm filter membrane (purchased from Millopore) for transfection.

### (2) Cell transfection:

(II) Cell transfection: 293E cells (purchased from Invitrogen) were cultured in Freestyle 293 expression medium (purchased from Invitrogen). The shaker was set to 37°C, 130RPM, and 8% CO₂ (v/v) concentration.

During transfection, Freestyle 293 expression medium was added with 10% (v/v) F68 (purchased from Invitrogen) to a final concentration of 0.1% (v/v), to obtain Freestyle 293 expression culture containing 0.1% (v/v) F68, that is, medium A.

5mL of medium A was taken and mixed well with 200µg/mL PEI (purchased from Sigma), to obtain medium B. 5 mL of medium A was taken and mixed well with 100µg/mL of the recombinant plasmid obtained in step (I) to obtain medium C. 5 minutes later, medium B and medium C were combined and mixed, and the mixture was let stand for 15 minutes to obtain a mixture D. 10mL of mixture D was slowly added into 100mL of Freestyle 293 expression medium containing 293E cells, until the cell density of 293E was 1.5×10⁶/mL, and it was shaked while being added, to avoid excessive aggregation of PEI. And the mixture was placed in a shaker for culture. Peptone was added the next day to a final concentration of 0.5% (w/v). On days 5-7, the antibody titer of the culture medium was measured. On days 6-7, the supernatant was collected by centrifugation (3500RPM, 30 minutes) and filtered through a 0.22µm filter to obtain the filtered cell supernatant for purification.

(III) Antibody purification: For continuouly used endotoxin-free chromatography columns and Protein A stuffing (purchased from GE), 0.1M NaOH was used for washing for 30 minutes, or 5 column volumes of 0.5M NaOH was used for washing. For long-term unused column materials and chromatography columns, at least 1M NaOH was used for soaking for 1 hour, and non-endotoxic water was used for rinsing to neutrality, and the column material was washed with 10 column volume of 1% (v/v) Triton×100. 5 column volumes of PBS (PBS phosphate buffer, pH 7.2) was used for equilibrate. The filtered cell supernatant obtained in step (II) was loaded on the column, and the flow-through liquid was collectd if necessary. After the samples were loaded, the column was washed with 5 column volume of PBS. Elution was performed with 5 column volumes of 0.1M pH3.0 Glycine-HCl, and the eluate was collected, and neutralized with 0.5 column volume of 1M Tris-HCl (1.5M NaCl) with pH 8.5. And fully human TIM-3 antibody was obtained. All the above-mentioned solutions required a fresh configuration. The fully human TIM-3 antibodies were harvested, and then dialyzed for 4 hours in 1×PBS to avoid endotoxin contamination. After dialysis, spectrophotometry or a kit was used to determine the concentration, and HPLC-SEC was used to determine the purity of the antibody, and an endotoxin detection kit (purchased from Lonza) was used to detect the content of antibody endotoxin.

### EXAMPLE 5 Identification of fully human TIM-3 antibody

### (I) Enzyme-linked immunosorbent assay (ELISA) detection of the binding of antibodies to TIM-3 protein

The results are shown in Figures 10, 11 and 12, Tables 10, 11 and 12. Wherein, the IgG control is human IgG, and the data in the table is A450nm values.

**Table 10 Activity of fully human TIM-3 antibodies reacting with human TIM-3 extracellular domain protein in the enzyme-linked immunosorbent assay.**

| Clone ID | OD450 | | | | | | | |
|---|---|---|---|---|---|---|---|---|
| | Antibody concentration (nM) | | | | | | | |
| | 66.667 | 6.6667 | 0.6667 | 0.0667 | 0.0067 | 0.0007 | 0.0001 | 0 |
| 7A4F10 | 3.02 | 3.01 | 2.75 | 1.69 | 0.32 | 0.11 | 0.08 | 0.08 |
| 18D2H2 | 2.78 | 2.61 | 2.58 | 1.57 | 0.32 | 0.11 | 0.08 | 0.07 |
| 34B6D8 | 3.10 | 2.99 | 3.11 | 1.58 | 0.29 | 0.11 | 0.09 | 0.09 |
| 39E5H1 | 3.01 | 2.95 | 3.01 | 1.33 | 0.25 | 0.10 | 0.08 | 0.08 |
| 57F4E5 | 3.06 | 2.91 | 2.89 | 1.22 | 0.23 | 0.11 | 0.08 | 0.08 |
| 134H3G6 | 2.77 | 2.81 | 2.74 | 1.28 | 0.27 | 0.10 | 0.10 | 0.08 |
| 215A8F2 | 3.08 | 2.86 | 2.88 | 1.29 | 0.27 | 0.10 | 0.08 | 0.08 |
| hIgG control | 0.14 | 0.08 | 0.07 | 0.07 | 0.07 | 0.07 | 0.07 | 0.07 |

**Table 11 Activity of fully human TIM-3 antibodies reacting with monkey TIM-3 extracellular domain protein in the enzyme-linked immunosorbent assay.**

| Clone ID | **OD450** | | | | | | | |
|---|---|---|---|---|---|---|---|---|
| | Antibody concentration (nM) | | | | | | | |
| | 66.667 | 6.6667 | 0.6667 | 0.0667 | 0.0067 | 0.0007 | 0.0001 | 0 |
| 7A4F10 | 3.26 | 3.12 | 2.46 | 0.68 | 0.18 | 0.14 | 0.13 | 0.13 |
| 18D2H2 | 3.27 | 3.30 | 3.16 | 1.56 | 0.33 | 0.15 | 0.14 | 0.13 |
| 34B6D8 | 3.39 | 3.39 | 3.37 | 1.71 | 0.37 | 0.19 | 0.17 | 0.17 |
| 39E5H1 | 3.38 | 3.35 | 3.23 | 1.37 | 0.30 | 0.18 | 0.17 | 0.16 |
| 57F4E5 | 3.42 | 3.29 | 3.15 | 1.34 | 0.32 | 0.18 | 0.17 | 0.16 |
| 134H3G6 | 3.11 | 3.19 | 3.10 | 1.30 | 0.30 | 0.15 | 0.14 | 0.13 |
| 215A8F2 | 3.24 | 3.28 | 3.05 | 1.37 | 0.33 | 0.15 | 0.13 | 0.13 |
| hIgG control | 0.43 | 0.18 | 0.15 | 0.13 | 0.13 | 0.14 | 0.13 | 0.13 |

**Table 12 Activity of fully human TIM-3 antibodies reacting with mouse TIM-3 extracellular domain protein in the enzyme-linked immunosorbent assay.**

| Clone ID | **OD450** | | | | | | | |
|---|---|---|---|---|---|---|---|---|
| | Antibody concentration (nM) | | | | | | | |
| | 66.667 | 6.6667 | 0.6667 | 0.0667 | 0.0067 | 0.0007 | 0.0001 | 0 |
| 7A4F10 | 0.99 | 0.36 | 0.25 | 0.22 | 0.23 | 0.22 | 0.22 | 0.23 |
| 18D2H2 | 0.81 | 0.31 | 0.22 | 0.22 | 0.22 | 0.21 | 0.21 | 0.22 |
| 34B6D8 | 1.38 | 0.44 | 0.27 | 0.25 | 0.24 | 0.24 | 0.23 | 0.24 |
| 39E5H1 | 1.28 | 0.41 | 0.26 | 0.24 | 0.23 | 0.23 | 0.23 | 0.24 |
| 57F4E5 | 1.55 | 0.54 | 0.28 | 0.33 | 0.23 | 0.23 | 0.24 | 0.24 |
| 134H3G6 | 0.94 | 0.36 | 0.23 | 0.21 | 0.22 | 0.21 | 0.22 | 0.22 |
| 215A8F2 | 1.08 | 0.38 | 0.24 | 0.31 | 0.22 | 0.21 | 0.21 | 0.21 |
| hIgG control | 0.73 | 0.30 | 0.22 | 0.21 | 0.21 | 0.21 | 0.22 | 0.23 |

The results show that the fully human TIM-3 antibody can bind to the extracellular domain protein of human and monkey TIM-3, but cannot bind to mouse TIM-3 protein. The activity of each antibody is equivalent, which indicates that the antibody has strong binding ability with TIM-3.

### (II) Detection of the binding of antibodies to TIM-3 expressing cells by Flow cytometry (FACS)

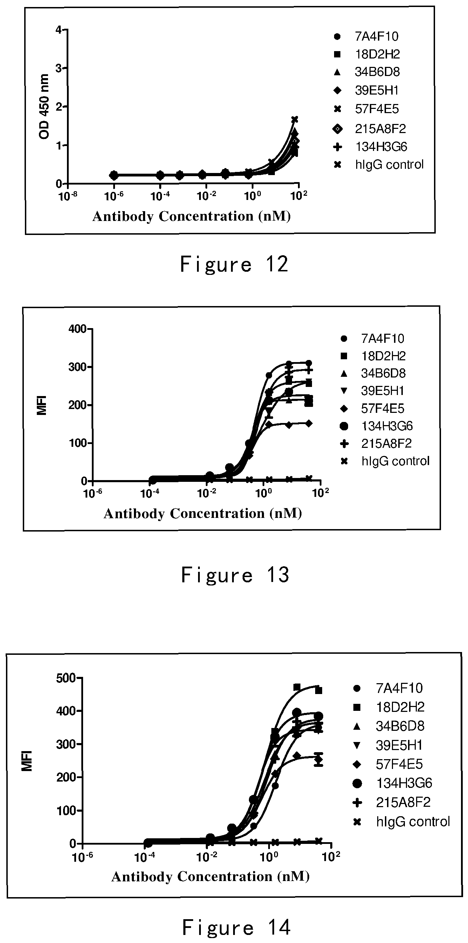

The results are shown in Figure 13 and Figure 14, Table 13 and Table 14. Wherein, the IgG control was human IgG, and the data in the table is the average fluorescence intensity values of the cell populations measured by MFI.

**Table 13 Binding reaction of fully human TIM-3 antibody to CHOK1-hTIM-3 detected by FACS**

| Clone ID | Mean fluorescence intensity | | | | | | |
|---|---|---|---|---|---|---|---|
| | Antibody concentration (nM) | | | | | | |
| | 40 | 8 | 1.6 | 0.32 | 0.064 | 0.013 | 0 |
| 7A4F10 | 312.8 | 307.6 | 282.3 | 90.1 | 28.8 | 9.7 | 2.8 |
| 18D2H2 | 256.7 | 267.5 | 232.4 | 93.1 | 32.9 | 11.6 | 2.8 |
| 34B6D8 | 220.9 | 218.9 | 211.5 | 76.9 | 22.2 | 9.2 | 2.9 |
| 39E5H1 | 263.7 | 233.7 | 190.7 | 73.1 | 27.0 | 9.2 | 2.8 |
| 57F4E5 | 147.0 | 154.3 | 155.5 | 69.7 | 23.7 | 9.6 | 2.9 |
| 134H3G6 | 218.3 | 229.0 | 207.2 | 96.6 | 34.5 | 13.5 | 2.8 |
| 215A8F2 | 283.1 | 297.9 | 235.3 | 73.6 | 20.5 | 8.2 | 2.9 |
| hIgG control | 5.0 | 4.5 | 3.1 | 2.9 | 2.8 | 2.8 | 2.8 |

**Table 14 Binding reaction of fully human TIM-3 antibody to CHOK1-cTIM-3 detected by FACS**

| Clone ID | Mean fluorescence intensity | | | | | | |
|---|---|---|---|---|---|---|---|
| | Antibody concentration (nM) | | | | | | |
| | 40 | 8 | 1.6 | 0.32 | 0.064 | 0.013 | 0 |
| 7A4F10 | 336.1 | 325.2 | 176.8 | 52.7 | 17.2 | 7.1 | 2.8 |
| 18D2H2 | 461.9 | 475.1 | 332.7 | 126.8 | 44.0 | 14.8 | 2.8 |
| 34B6D8 | 336.2 | 340.0 | 265.9 | 108.6 | 35.4 | 12.7 | 2.9 |
| 39E5H1 | 336.9 | 348.8 | 315.1 | 131.8 | 42.3 | 14.8 | 3.0 |
| 57F4E5 | 269.2 | 258.6 | 207.0 | 80.6 | 30.6 | 11.4 | 2.9 |
| 134H3G6 | 376.1 | 400.9 | 326.0 | 133.9 | 46.4 | 16.8 | 2.9 |
| 215A8F2 | 365.8 | 362.4 | 251.5 | 92.5 | 29.9 | 11.5 | 2.8 |
| hIgG control | 6.0 | 3.7 | 3.2 | 3.1 | 2.9 | 2.8 | 2.9 |

The results show that the fully human TIM-3 antibody can bind to human and monkey TIM-3 proteins on the cell surface to varying degrees.

### (III) Detection of TIM-3 antibody blocking of the binding of TIM-3 to its ligand phosphatidylserine by TIM-3 receptor ligand binding assay

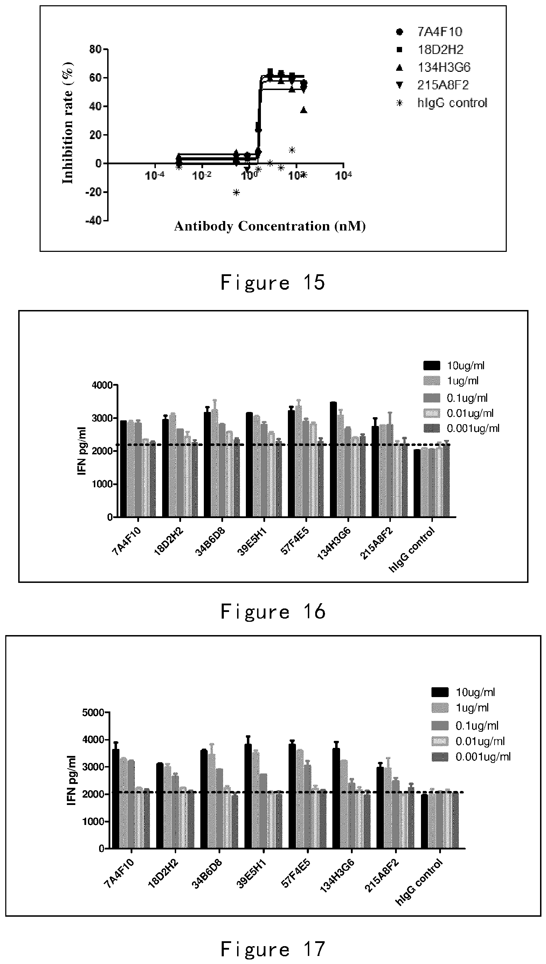

Jurkat cells were treated with 1 µM staurosporine at 37 °C for 2 hours to induce apoptosis. The presence of phosphatidylserine was detected by Annexin V-FITC cell apoptosis detection kit. The hTIM-3-hFc strongly bound to staurosporine-treated Jurkat cells, but did not bind to untreated Jurkat cells. The cells were washed 1-2 times with PBS buffer, and after cell counting, the cells were diluted to 1-2 × 10⁶ cells per milliliter with binding buffer, and added to a 96-well FACS reaction plate at 100 microliters per well. The antibody sample to be tested and 2 µg/ml hTIM-3-hFc protein were prepared with binding buffer, and mixed equally in volume. The mixture was incubated at room temperature for 30 minutes, then the FACS reaction plate incubated with apoptotic cells was centrifuged to discard the supernatant, and the above mixture was added to the apoptotic cells at 100 microliters per well and incubated at room temperature for 1 hour. The plate was washed twice with the binding buffer by centrifugation, added with 100 microliters of fluorescent (Alexa 488)-labeled secondary antibodies per well, and incubated for half an hour. The plate was washed 2-3 times with the binding buffer by centrifugation, added with 100 microliters of PBS per well to suspend cells, and detected using FACS (FACS Calibur, BD) and the results were analyzed. The results are shown in Table 15 and Figure 15, wherein the IgG control is human IgG, and the data in the table is the inhibition rate (%).

**Table 15 Fully human TIM-3 antibody blocks binding of TIM-3 protein to its receptor phosphatidylserine**

| Inhibition ratio (%) | Antibody concentration (nM) | | | | | | | |
|---|---|---|---|---|---|---|---|---|
| Clone number | 200 | 66.667 | 22.222 | 7.407 | 2.469 | 0.823 | 0.274 | 0 |
| 7A4F10 | 56.9 | 60.5 | 62.1 | 62.4 | 23.7 | 5.7 | 0.9 | 0.5 |
| 18D2H2 | 56.1 | 62.3 | 63.9 | 64.9 | 27.4 | 3.9 | 5.4 | 1.4 |
| 134H3G6 | 37.6 | 52.3 | 58.4 | 60.1 | 10.9 | 6.1 | 8.0 | 5.5 |
| 215A8F2 | 52.1 | 57.2 | 61.8 | 59.5 | 5.8 | -4.4 | 0.3 | 1.6 |
| hIgG control | -7.8 | 9.6 | -2.8 | 0.5 | -3.9 | 2.8 | -20.0 | -2.5 |

The results show that the antibody can inhibit the binding of TIM-3 protein to its receptor phosphatidylserine to varying degrees, and the activity of the antibody was equivalent.

### (IV) Detection of the effect of TIM-3 antibody on lymphocyte activity by lymphocyte stimulation test

In the lymphocyte stimulation test, TIM-3 antibodies block the binding of TIM-3 protein and its receptor to relieve the inhibition of T lymphocyte activity, thereby stimulating the proliferation of T cells.

Results are shown in Figures 16 and 17, Tables 16 and 17, PBMC cells from two donors were selected in this experiment, and the results were basically consistent. Wherein, the hIgG control is human IgG, and the data in the table is IFN-γ value (pg/mL).

**Table 16 Effect of fully human TIM-3 antibodies on IFN-γ secretion in lymphocyte activation assay (donor X)**

| Clone ID | IFN-γ production, pg/ml | | | | | | | | | |
|---|---|---|---|---|---|---|---|---|---|---|
| | 10µg/ml | | 1µg/ml | | 0.1µg/ml | | 0 | | 0 | |
| | N=1 | N=2 | N=1 | N=2 | N=1 | N=2 | N=1 | N=2 | N=1 | N=2 |
| 7A4F10 | 2904.8 | 2356.6 | 2922.9 | 2784.1 | 2933.9 | 2732.6 | 2338.1 | 2365.3 | 2289.5 | 2230.5 |
| 18D2H2 | 2809.9 | 3074.1 | 3003.0 | 3137.4 | 2659.2 | 2647.8 | 2282.7 | 2587.8 | 2335.2 | 2165.8 |
| 34B6D8 | 2977.2 | 3331.7 | 2930.3 | 3549.0 | 2772.6 | 2818.4 | 2598.7 | 2541.4 | 2281.4 | 2394.9 |
| 39E5H1 | 3153.1 | 3127.9 | 3071.7 | 3004.5 | 2699.5 | 2881.6 | 2581.1 | 2459.7 | 2193.1 | 2372.7 |
| 57F4E5 | 3078.7 | 3341.3 | 3545.5 | 3148.9 | 2800.7 | 2983.1 | 2870.2 | 2741.8 | 2158.9 | 2395.5 |
| 134H3G6 | 3462.7 | 3476.0 | 3251.9 | 2894.0 | 2606.8 | 2724.7 | 2386.8 | 2425.9 | 2506.7 | 2365.6 |
| 215A8F2 | 2997.2 | 2471.5 | 2779.9 | 2031.9 | 2409.1 | 3167.6 | 2098.3 | 2302.3 | 1974.4 | 2397.0 |
| hIgG1 Control | 2034.4 | 2020.2 | 2091.5 | 2072.5 | 2040.8 | 2063.9 | 2260.5 | 1900.1 | 2003.8 | 2315.0 |

**Table 17 Effect of fully human TIM-3 antibodies on IFN-γ secretion in lymphocyte activation assay (donor Y)**

| Clone ID | IFN-γ production, pg/ml | | | | | | | | | |
|---|---|---|---|---|---|---|---|---|---|---|
| | 10µg/ml | | 1µg/ml | | 0.1µg/ml | | 0 | | 0 | |
| | N=1 | N=2 | N=1 | N=2 | N=1 | N=2 | N=1 | N=2 | N=1 | N=2 |
| 7A4F10 | 3341.3 | 3891.2 | 3325.1 | 3219.3 | 3154.5 | 3224.3 | 2248.1 | 2157.7 | 2176.4 | 2089.1 |
| 18D2H2 | 3048.9 | 3126.2 | 3106.3 | 2864.8 | 2540.8 | 2750.3 | 2203.9 | 2235.7 | 2125.7 | 2051.7 |
| 34B6D8 | 3515.2 | 3631.6 | 3045.6 | 3825.5 | 2887.5 | 2899.5 | 2127.3 | 2304.1 | 1823.3 | 2043.8 |
| 39E5H1 | 3497.1 | 4116.1 | 3401.2 | 3599.3 | 2712.9 | 2712.9 | 2094.1 | 1951.0 | 1843.7 | 2101.2 |
| 57F4E5 | 3960.3 | 3661.6 | 3547.3 | 3612.7 | 3214.3 | 2866.6 | 2310.4 | 2048.6 | 2151.4 | 2096.7 |
| 134H3G6 | 3403.1 | 3910.9 | 3196.6 | 3226.0 | 2215.4 | 2546.4 | 2025.1 | 2256.6 | 1806.4 | 2121.3 |
| 215A8F2 | 3141.5 | 2791.8 | 3321.2 | 2556.3 | 2598.1 | 2341.0 | 1991.1 | 1996.8 | 2381.0 | 2071.5 |
| hIgG control | 1926.8 | 1982.5 | 2186.2 | 1769.0 | 2097.4 | 2016.0 | 1985.2 | 2160.5 | 1997.4 | 2068.7 |

The results show that the antibody to be tested can enhance IFN-γ secretion of PBMC to varying degrees in PBMC lymphocyte stimulation test.

### (V) Antibody affinity test

First, AHC sensor was selected, and the sensor was balanced with buffer solution for 10min, then the antibody to be tested was diluted to 5 µg/ml with buffer solution, and the antibody was solidified with the sensor for 3-5min, with a signal value of 1-2nm. The sensor was balanced again with buffer solution for 3 minutes, and then the antigen protein was diluted to 100nM (the highest concentration was tentatively 100nM) to bind and dissociate the antibody coupled by the sensor. If enough signal value was obtained, the antigen protein was diluted by several concentration gradients to analyze the binding and dissociation of antibody and antigen. At that end of each cycle, the sensor surface was regenerated with 10mM, pH 1.5 Glycine. The kinetic rate constant needed to be subtracted the blank control, and the data was fitted with the global fit analysis method 1:1 combination model. The dissociation equilibrium rate constant (KD) was calculated according to the following formula: KD=kd/ka. The results are shown in Table 18.

**Table 18 Analysis and determination of anti-TIM-3 antibody affinity**

| Clone ID | Ka (1/Ms) | Kd (1/s) | KD (M) |
|---|---|---|---|
| 7A4F10 | 1.11E+06 | 1.13E-03 | 1.01E-09 |
| 18D2H2 | 1.22E+06 | 3.13E-03 | 2.58E-09 |
| 34B6D8 | 1.15E+06 | 1.20E-03 | 1.05E-09 |
| 39E5H1 | 1.06E+06 | 1.55E-03 | 1.46E-09 |
| 57F4E5 | 1.60E+06 | 3.97E-03 | 2.48E-09 |
| 134H3G6 | 1.19E+06 | 4.83E-03 | 4.05E-09 |
| 215A8F2 | 9.65E+05 | 1.28E-04 | 1.33E-10 |

The results show that the KD values of the obtained fully human TIM-3 antibodies were all at the level of nanomolecular (nM) level, indicating that these antibodies had good affinity to human TIM-3 ECD. Among them, 215A8F2 antibody had the best affinity for human TIM-3 ECD.

### Discussion

### Fully human antibody

The application of monoclonal antibodies is one of the most successful and transformative treatments in cancer treatment in the past 20 years. Compared with traditional chemical drugs, antibody drugs have higher specificity and lower toxicity. Although monoclonal antibody drugs have achieved continuous success, they still face many challenges.

The biggest defect of a murine monoclonal antibody is the HAMA (human anti-mouse antibody) response it induces. Therefore, murine monoclonal antibodies have great limitations in the diagnosis and treatment of tumors, organ transplantation and other diseases; chimeric antibodies still retain 30% of the murine sequence, which can cause different degrees of HAMA response. Clinically, different chimeric antibodies have different degrees of immunogenicity; humanized antibodies are also called transplantation antibodies. Simple CDR transplantation often leads to a decrease in antigen-antibody affinity. Because it still has at least 10% heterologous protein, its clinical application is still limited to varying degrees. Therefore, it is necessary to further develop a more perfect therapeutic antibody, a fully human antibody.

In 1994, the American companies Abgenix and Genpham reported the use of transgenic mice to prepare fully human antibodies, thus solving the problem of human antibody preparation research that the human body cannot be immunized at will. Since then, the technology for preparing fully human antibodies has continued to develop and mature, and the obtained human monoclonal antibodies have strong anti-tumor activity. H2L2 transgenic mouse is provided under license by Harbour Medicine (Shanghai) Co., Ltd., based on the transgenic mouse technology developed by Professor Frank Grosveld's laboratory of Rotterdam University Medical Center (Rotterdam, Netherlands), which can produce a traditional tetrameric antibody consisting of two heavy chains and two light chains (H2L2) with complete human variable regions. The antibodies produced have mature affinity, fully humanized variable regions, and have excellent solubility. Genetically engineered mouse technology is one of the main tools for producing fully human antibodies.

### Immunotherapy

Cancer immunotherapy refers to a treatment that uses the immune system to fight cancer. Recently, cancer immunotherapy has attracted much attention. It has become a new method of cancer treatment besides surgery, chemotherapy and radiotherapy. Immune checkpoints refer to some inhibitory signal pathways in the immune system, which prevent tissue damage by regulating the persistence and intensity of immune responses in peripheral tissues, and participate in maintaining tolerance to self-antigens. The use of inhibitory signaling pathways of immune checkpoints to inhibit T cell activity is an important mechanism for tumors to escape immune killing. Blocking immune checkpoints is one of many effective strategies to activate anti-tumor immunity.

Inhibitors of immune checkpoint proteins have the potential to treat various tumor types (such as metastatic melanoma, lung cancer, breast cancer, renal cell carcinoma, etc.). Recent research on cancer immunotherapy has shown promising results, especially for metastatic cancer cases. In addition, cancer immunotherapy has great potential in the treatment of blood cancers, including Hodgkin's lymphoma, multiple myeloma, myelodysplastic syndrome, and non-Hodgkin's lymphoma. The side effects caused by immune checkpoint inhibitors are negligible, reversible and controllable, and effective immune checkpoint inhibitors can significantly improve the overall survival of cancer patients. Immune checkpoint inhibitors can also be used in combination with targeted therapy or conventional radiotherapy and chemotherapy, and this combination therapy can effectively treat many types of cancer, and may be the hope of treating or curing a variety of cancers.

At present, the clinical research of TIM-3 antibody is mainly used in the treatment of malignant solid tumor and lymphoma, and it is also mainly concentrated on its combined use with other therapies or target drugs to develop antibodies with a wide range of indications to expand its applicable clinical symptoms, including unresectable metastatic melanoma, advanced solid cancer, breast cancer, endometrial cancer, ovarian cancer, kidney cancer, pancreatic cancer, recurrent glioblastoma, head and neck cancer, bladder cancer, metastasis rectal cancer, gastrointestinal stromal tumors, acinar cell carcinoma, high-grade malignant solid tumors, non-small cell lung cancer, etc.

The technical scheme of the present invention has the advantage that:
Therapeutic monoclonal antibodies can be developed by various technologies and approaches, including hybridoma technology, phage display technology, and single lymphocyte gene cloning technology and so on. However, the preparation of monoclonal antibodies from wild-type or transgenic mice by hybridoma technology is still the mainstream of the preparation methods of therapeutic monoclonal antibodies. According to the latest advances in monoclonal antibody technology, the present invention adopts optimized hybridoma technology to prepare the required anti-TIM-3 antibody.
(1) Antibodies can be obtained by immunizing wild-type mice, but mouse antibodies need to be humanized to obtain humanized antibodies. The disadvantage is that the modified antibody may be more immunogenic and the structure of the antibody may be changed resulting in loss of activity or poor manufacturability.
(2) Antibodies can obtained by immunizing fully human transgenic mice, but the number or affinity of the obtained antibodies will be poor.
(3) Antibody expression and activity screening can be carried out by constructing immunized mouse antibody library with phage display technology, but the random recombination of antibody heavy and light chains will result in poor production of the formed antibodies.
(4) Antibodies can be expressed and screened by phage display technology by constructing a human antibody library. But the affinity of the obtained antibody will be poor because it has not been immunized.

All literatures mentioned in the present application are incorporated herein by reference, as though each one is individually incorporated by reference. In addition, it should also be understood that, after reading the above teachings of the present invention, those skilled in the art can make various changes or modifications, equivalents of which falls in the scope of claims as defined in the appended claims.

The sequence information of the present invention (including the amino acid sequence of the CDR region in the TIM-3 antibody of the present invention and its sequence number, and the amino acid and gene sequence of the heavy/light chain variable region in the TIM-3 antibody of the present invention and its sequence number) is shown in Table 18 below.

**Table 18**

| SEQ ID NO. | Name | Sequence |
|---|---|---|
| 1 | 7A4F10 VH-aa | EVQLLESGGGLVQPGGSLRLSCAASGFTFRTHLISWVRQAPGKGLEWVAAITGSG GSTYYADSVKGRFIISRDNSKNTLFLQMNSLRAEDTAVYYCAKDGGSGSYDDFW GQGTLVTVSS |
| 2 | 7A4F10 VH-nt | gaagtgcaactgttggagtctgggggaggcttggtacagcctggggggtccctgagactctcctgtgcggcctctggattcac ctttaggacccatctcataagttgggtccgccaggctccagggaagggactggagtgggtcgcagctataactggtagtggt ggtagcacatactacgcagactccgtgaagggccggttcatcatctccagagacaattccaagaacacgctgtttctgcaaat gaacagcctgagagccgaagacacggccgtatattactgtgcgaaagatggggggttcggggagttatgatgacttctggggc cagggaaccctggtcaccgtctcctca |
| 3 | 7A4F10 VH-CDR1 | THLIS |
| 4 | 7A4F10 VH-CDR2 | AITGSGGSTYYADSVKG |
| 5 | 7A4F10 VH-CDR3 | DGGSGSYDDF |
| 6 | 7A4F10 VL-aa | ETVLTQSPVTLSLSPGERATLSCRASQSVGSYLAWYQQKPGQAPRLLIYDASNRA TGIPARFSGSGSGTDFTLTISSLEPEDFAVYYCQQRSNWPPTFGQGTKVEIK |
| 7 | 7A4F10 VL-nt | gaaactgtgttgacacagtctccagtcaccctgtctttgtctccaggggaaagagccaccctctcctgcagggccagtcagag tgttggcagctacttagcctggtaccaacagaaacctggccaggctcccaggctcctcatctatgatgcatccaacagggcca ctggcatcccagccaggttcagtggcagtgggtctgggacagacttcactctcaccatcagcagcctagagcctgaagatttt gcagtttattactgtcagcagcgtagcaactggcctccgacgttcggccaagggaccaaggtggaaatcaaa |
| 8 | 7A4F10 VL-CDR1 | RASQSVGSYLA |
| 9 | 7A4F10 VL-CDR2 | DASNRAT |
| 10 | 7A4F10 VL-CDR3 | QQRSNWPPT |
| 11 | 18D2H2 VH-aa | EVQLVESGGDLVQPGGSLRLSCAASGFTFSTYWMTWVRQAPGRGLEWVANIKQ DGSAKYYVDSVKGRFTLSRDDAKNSLYLQMNSLRAEDTAVYYCARGSNWFDY WGQGTLVTVSS |
| 12 | 18D2H2 VH-nt | gaggtgcagctggtggagtctgggggagacttggtccagcctggggggtccctgagactctcctgtgcagcctctggattca cctttagtacctattggatgacgtgggtccgccaggctccagggaggggactggagtgggtggccaacattaaacaagatgg aagtgcgaaatactatgtggactctgtgaagggccgattcaccctctccagagacgacgccaagaactcactgtatctgcaaa tgaacagcctgagagccgaggacacggctgtgtattactgtgcgagagggagcaactggtttgactactggggccagggaa ccctggtcaccgtctcctca |
| 13 | 18D2H2 VH-CDR1 | TYWMT |
| 14 | 18D2H2 VH-CDR2 | NIKQDGSAKYYVDSVKG |
| 15 | 18D2H2 VH-CDR3 | GSNWFDY |
| 16 | 18D2H2 VL-aa | ETVMTQSPATLSVSPGERATLSCRANQNVYSNLAWYQQKPGQAPRLLIYDASTR ATGFPARFSGSGSGTEFTLTISSLQSEDFAVYYCQQYNNWPLTFGGGTKVEIK |
| 17 | 18D2H2 VL-nt | gaaacagtgatgacgcagtctccagccaccctgtctgtgtctccaggggaaagagccaccctctcctgcagggccaatcaga atgtttacagcaacttagcctggtaccagcagaaacctggccaggctcccaggctcctcatctatgatgcatccaccagggcc actggtttcccagccaggttcagtggcagtgggtctgggacagagttcactctcaccatcagcagcctgcagtctgaagatttt gcagtttattactgtcagcagtataataactggccgctcactttcggcggagggaccaaggtggagatcaaa |
| 18 | 18D2H2 VL-CDR1 | RANQNVYSNLA |
| 19 | 18D2H2 VH-CDR2 | DASTRAT |
| 20 | 18D2H2 VH-CDR3 | QQYNNWPLT |
| 21 | 134H3G6 VH-aa | EVQLVESGGGLVQPGGSLRLSCAASGFTFSNSWMTWVRQAPGKGLEWVANIKQ AGTEKYYVDSVKGRFTISRDNAKNSLYLQMNSLRAEDTAVYYCNSGSNYFDYW GQGTLVTVSS |
| 22 | 134H3G6 VH-nt | gaggtgcagctggtggagtctgggggaggcttggtccagcctggggggtccctgagactctcctgtgcagcctctggattca cctttagtaactcttggatgacctgggtccgccaggctccagggaaggggctggagtgggtggccaacataaagcaagctg gaactgagaaatactatgtggactctgtgaagggccgattcaccatctccagagacaacgccaagaactcactgtatctgcaa atgaacagcctgagagccgaggacacggctgtgtattactgtaatagtgggagcaactactttgactactggggccagggaa ccctggtcaccgtctcctca |
| 23 | 134H3G6 VH-CDR1 | NSWMT |
| 24 | 134H3G6 VH-CDR2 | NIKQAGTEKYYVDSVKG |
| 25 | 134H3G6 VH-CDR3 | GSNYFDY |
| 26 | 134H3G6 VL-aa | EIVMTQSPATLSVSPGERATLSCRARQNFYSNLAWYQQKPGQAPRLLIYGASTRA TGIPARFSGSGSGTEFTLTISSLQSEDFAVYYCQQYNNWPLTFGGGTKVEIK |
| 27 | 134H3G6 VL-nt | gaaatagtaatgacgcagtctccagccaccctgtctgtgtctccaggggaaagagccaccctctcctgcagggccagacaga atttttacagcaacttagcctggtaccaacagaaacctggccaggctcccaggctcctcatctatggtgcatccaccagggcca ctggtatcccagccaggttcagtggcagtgggtctgggacagagttcactctcaccatcagcagcctgcagtctgaagattttg cagtttattactgtcagcagtataataactggcctctcactttcggcggagggaccaaggtggagatcaaa |
| 28 | 134H3G6 VL-CDR1 | RARQNFYSNLA |
| 29 | 134H3G6 VL-CDR2 | GASTRAT |
| 30 | 134H3G6 VL-CDR3 | QQYNNWPLT |
| 31 | 215A8F2 VH-aa | QVQLQQWGAGLLKPSETLSLTCTVYGGSFSGYYWNWIRQPPGKGLEWLGEINHS GSTNYNPSLKRRVTISVDTSKNQFSLKLSSVTAADTAVYYCARVYYYGPFDFWG QGTLVTVSS |
| 32 | 215A8F2 VH-nt | caggtgcagctacagcagtggggcgcaggactgttgaagccttcggagaccctgtccctcacctgcactgtctatggtgggt ccttcagtggttactactggaactggatccgccagcccccagggaaggggctggagtggcttggggaaatcaatcatagtgg aagcaccaactacaacccgtccctcaagagacgagtcaccatatcagtggacacgtccaagaaccagttctccctgaagctg agctctgtgaccgccgcggacacggctgtgtattactgtgcgagggtgtattattatggcccttttgacttctggggccaggga accctggtcaccgtctcctca |
| 33 | 215A8F2 VH-CDR1 | GYYWN |
| 34 | 215A8F2 VH-CDR2 | EINHSGSTNYNPSLKR |
| 35 | 215A8F2 VH-CDR3 | VYYYGPFDF |
| 36 | 215A8F2 VL-aa | DIVMTQSPDSLAVSLGERATINCKSSQSVLYRSNNKNYLAWYQQKPGQPPKLLIY WASTRESGVPDRFSGSGPGTDFTLTINSLQAEDVAVYYCQQYYSTPYTFGQGTKL EIK |
| 37 | 215A8F2 VL-nt | gacatcgtgatgacccagtctccagactccctggctgtgtctctgggcgagagggccaccatcaactgcaagtccagccaga gtgttttatacaggtccaacaataagaactacttagcttggtaccagcagaaaccaggacagcctcctaagctgctcatttactg ggcatctacccgggaatccggggtccctgaccgattcagtggcagcgggcctgggacagatttcactctcaccatcaacagc ctgcaggctgaagatgtggcagtttattactgtcagcaatattatagtactccgtacacttttggccaggggaccaagctggaga tcaaa |
| 38 | 215A8F2 VL-CDR1 | KSSQSVLYRSNNKNYLA |
| 39 | 215A8F2 VL-CDR2 | WASTRES |
| 40 | 215A8F2 VL-CDR3 | QQYYSTPYT |
| 41 | 34B6D8 VH-aa | EVQLVESGGGLVQPGGSLRLSCAASGFTFSTYWMTWVRQAPGKRLEWVANIKQ GGGDKYYVDSVKGRFTISTDNAKNSLYLQMNSLRAEDTAVYYCARGSNWFDPW GQGTLVTVSS |
| 42 | 34B6D8 VH-nt | gaggtgcagctggtggagtctgggggaggcttggtccagcctggggggtccctgcgtctctcctgtgcagcctctggattca cctttagtacctattggatgacctgggtccgccaggctccagggaagcggctggagtgggtggccaacataaagcaaggtg gaggtgataaatactatgtggactctgtgaagggccgattcaccatctccacagacaacgccaagaactcactgtatctgcaa atgaacagcctgagagccgaggacacggctgtgtattactgtgcgagagggtccaactggttcgaccccctggggccaggga accctggtcaccgtctcctca |
| 43 | 34B6D8 VH-CDR1 | TYWMT |
| 44 | 34B6D8 VH-CDR2 | NIKQGGGDKYYVDSVKG |
| 45 | 34B6D8 VH-CDR3 | GSNWFDP |
| 46 | 34B6D8 VL-aa | EIVMTQSPATLSMSPGERATLSCRASQSVYDNLAWYQQKPGQAPRLLIYGASTR ATGIPARFSGSGSGTEFTLTISSLQSEDFALYYCQQYTHWPITFGQGTRLEIK |
| 47 | 34B6D8 VL-nt | gaaatagtgatgacgcagtctccagccaccctgtctatgtctccaggggaaagagccaccctctcctgcagggccagtcaga gtgtttacgacaacttagcctggtaccagcagaaacctggccaggctcccaggctcctcatctatggtgcatccaccagggcc actggtatcccagccaggttcagtggcagtgggtctgggacagagttcactctcaccatcagcagcctgcagtctgaagatttt gcactttattactgtcagcagtatactcactggccgatcaccttcggccaagggacacgactggagattaaa |
| 48 | 34B6D8 VL-CDR1 | RASQSVYDNLA |
| 49 | 34B6D8 VL-CDR2 | GASTRAT |
| 50 | 34B6D8 VL-CDR3 | QQYTHWPIT |
| 51 | 39E5H1 VH-aa | EVQLVESGGGLVQPGGSLRLSCAASGFTFSSYWMSWVRQAPGKGLEWVANIKR GGSERYYVDSVKGRFTISGDNAKNSVYLQMNSLRAEDTAVYYCARGSNWFDPW GQGTLVTVSS |
| 52 | 39E5H1 VH-nt | gaggtgcagctggtggagtctgggggaggcttggtccagcctggggggtccctgagactctcctgtgcagcctctggattca cctttagtagctattggatgagctgggtccgccaggctccagggaagggactggagtgggtggccaacataaagcgaggtg gaagtgagagatactatgtggactctgtgaagggccgattcaccatctccggagacaacgccaagaactcagtgtatctgcaa atgaacagcctgagagccgaggacacggctgtgtattactgtgcgagaggctccaactggttcgacccctggggccaggga accctggtcaccgtctcctca |
| 53 | 39E5H1 VH-CDR1 | SYWMS |
| 54 | 39E5H1 VH-CDR2 | NIKRGGSERYYVDSVKG |
| 55 | 39E5H1 VH-CDR3 | GSNWFDP |
| 56 | 39E5H1 VL-aa | ETVMTQSPAILSVSPGERATLSCRASQSVYSNLAWYQQKLGQAPRLLIYDASTRA TGIPARFSGSGSGTEFTLTISSLQSEDFAVYYCQQYNNWPITFGQGTRLEIK |
| 57 | 39E5H1 VL-nt | gaaacagtgatgacgcagtctccagccatcctgtctgtgtctccaggggaaagagccaccctctcctgcagggccagtcaga gtgtttacagcaacttagcctggtaccaacagaaacttggccaggctcccaggctcctcatctatgatgcatccaccagggcc actggtatcccagccaggttcagtggcagtgggtctgggacagagttcactctcaccatcagcagcctgcagtctgaagatttt gcagtttattactgtcagcagtataataactggccgatcaccttcggccaagggacacgactggagattaaa |
| 58 | 39E5H1 VL-CDR1 | RASQSVYSNLA |
| 59 | 39E5H1 VL-CDR2 | DASTRAT |
| 60 | 39E5H1 VL-CDR3 | QQYNNWPIT |
| 61 | 57F4E5 VH-aa | EVQLVESGGGLVQPGGSLRLSCAASGFTFSSYWMSWVRQAPGKGLEWVANIKQ DGSEKYYVDSVKGRFTISRDSAKNSLYLQMNSLRAEDTAMYFCARDSNFFDYW GQGTLVTVSS |
| 62 | 57F4E5 VH-nt | gaggtgcagctggtggagtctgggggaggcttggtccagcctggggggtccctgagactctcctgtgcagcctctggattca cctttagtagctattggatgagctgggtccgccaggctccagggaaggggctggagtgggtggccaacataaagcaagatg ggagtgagaaatactatgtggactctgtgaagggccgattcaccatctccagagacagcgccaagaactcactgtatcttca atgaacagcctgagagccgaggacacggctatgtatttctgtgcgagagactccaacttctttgactactggggccagggaa cctggtcaccgtctcctca |
| 63 | 57F4E5 VH-CDR1 | SYWMS |
| 64 | 57F4E5 VH-CDR2 | NIKQDGSEKYYVDSVKG |
| 65 | 57F4E5 VH-CDR3 | DSNFFDY |
| 66 | 57F4E5 VL-aa | ETVMTQSPATLSVSPGERATLSCRASQSVSSNLAWYQRKPGQAPRLLIYFSSTRA TGIPARFSGSGSGTEFTLTISSLQSEDFAVYYCQQYNNWPLTFGGGTKVEIK |
| 67 | 57F4E5 VL-nt | gaaacagtgatgacgcagtctccagccaccctgtctgtgtctccaggggaaagagccaccctctcctgcagggccagtcaga gtgttagcagcaacttagcctggtaccagcggaaacctggccaggctcccaggctcctcatctatttttcatccaccagggcca ctggaatcccagccaggttcagtggcagtgggtctgggacagagttcactctcaccatcagcagcctgcagtctgaagatttt gcagtttattactgtcagcagtataataactggccgctcactttcggcggagggaccaaggtggagatcaaa |
| 68 | 57F4E5 VL-CDR1 | RASQSVSSNLA |
| 69 | 57F4E5 VL-CDR2 | FSSTRAT |
| 70 | 57F4E5 VL-CDR3 | QQYNNWPLT |
| 71 | Amino acid sequence of human TIM-3 protein | MFSHLPFDCVLLLLLLLTRSSEVEYRAEVGQNAYLPCFYTPAAPGNLVPVCWGKGAC PVFECGNVLRTDERDVNYWTSRYWLNGDFRKGDVSLTIENVTLADSGIYCCRIQIPGI MNDEKFNLKLVIKPAKVTPAPTRQRDFTAAFPRMLTTRGHGPAETQTLGSLPDINLT QISTLANELRDSRLANDLRDSGATIRIGIYIGAGICAGLALIFKWYSHSKEKIQNLSLISL PPSGLANAVAEGIRSE ENIYTIEENVYEVEEPNEYYCYVSSRQQPSQPLGCRFAMP |
| 72 | Amino acid sequence of monkey TIM-3 protein | MFSHLPFDCVLLLLLLLTRSSEVEYIAEVGQNAYLPCSYTPAPPGNLVPVCWGKGAC PVFDCSNVVLRTDNRDVNDRTSGRYWLKGDFHKGDVSLTIENVTLADSGVYCCRIQI PGIMNDEKHNVKLVVIKPAKVTPAPTLQRDLTSAFPRMLTTGEHGPAETQTPGSLPD VNLTVSNFFCELQIFTLTNELRDSGATIRTAIYIAAGISAGLALIFGALIFKWYSHSKEK TQNLSLISLANIPPSGLANAVE AEGIRSEENIYTIEDVYEVEEPNEYYCYVSSGQQPSQPLGCRVAMP |
| 73 | Amino acid sequence of mouse TIM-3 protein | MFSGLTLNCVLLLLQLLLARSLEDGYKVEVGKNAYLPCSYTLPTSGTLVSYTLPTSGT LVPMCWGKGFCPWSQCTNELLRTDERNVTYQKSSRYQLKGDLKDLDVSLIIKNVTL |
| | | DDHGTYCCRIQFPGLMNDKKLELKLDIKAAKVTPAQTAHGDSTTASPRTTLTTERNG SETQTLVTLHNNNGTKISTWADEIKDSGETIRTAHIGVGVSAGLTLALIIGVLILKWYS CKKKLSSSLITLANLPPGGLANAG AVRIRSEENIYTIEENVYEVENSNEYYCYVNSQQPS |

| | | |
|---|---|---|
| Note: 1. Wherein VH-CDR1 is heavy chain variable region-CDRl, VH-CDR2 is heavy chain variable region-CDR2, and VH-CDR3 is heavy chain variable region-CDR3; wherein, VL-CDR1 is light chain variable region-CDRl, VL-CDR2 is light chain variable region-CDR2, and VL-CDR3 is light chain variable region-CDR3. 2. VH-aa refers to the amino acid sequence of the heavy chain variable region, and VH-nt refers to the gene sequence of the heavy chain variable region; VL-aa refers to the amino acid sequence of the light chain variable region, and VL-nt refers to the gene sequence of the heavy chain variable region. | | |

## Claims

1. A heavy chain variable region of an antibody, wherein the heavy chain variable region comprises the following three complementarity determining regions or CDRs:
VH-CDR1 shown in SEQ ID NO. 10n+3,
VH-CDR2 shown in SEQ ID NO. 10n+4, and
VH-CDR3 shown in SEQ ID NO. 10n+5;
wherein each n is independently 0, 1, 2, 3, 4, 5 or 6;
wherein any one of the above amino acid sequences further comprises a derivative sequence which is obtained through optional addition, deletion, modification and/or substitution of at least one amino acid and is capable of retaining the binding affinity to TIM-3.

2. A heavy chain of an antibody, wherein the heavy chain comprises the heavy chain variable region of claim 1.

3. A light chain variable region of an antibody, wherein the light chain variable region comprises the following three complementarity determining regions or CDRs:
VL-CDR1 shown in SEQ ID NO. 10n+8,
VL-CDR2 shown in SEQ ID NO. 10n+9, and
VL-CDR3 shown in SEQ ID NO. 10n+10;
wherein each n is independently 0, 1, 2, 3, 4, 5 or 6;
wherein any one of the above amino acid sequences further comprises a derivative sequence which is obtained through optional addition, deletion, modification and/or substitution of at least one amino acid and is capable of retaining the binding affinity to TIM-3.

4. A light chain of an antibody, wherein the light chain comprises the light chain variable region of claim 3.

5. An antibody, wherein the antibody comprises:
(1) the heavy chain variable region of claim 1; and/or
(2) the light chain variable region of claim 3;
or the antibody comprises: the heavy chain of claim 2; and/or the light chain of claim 4,
wherein any one of the above amino acid sequences further comprises a derivative sequence which is obtained through optional addition, deletion, modification and/or substitution of at least one amino acid and is capable of retaining the binding affinity to TIM-3.

6. The antibody of claim 5, wherein the antibody comprises the heavy chain variable region of claim 1 and the light chain variable region of claim 3;
wherein, the heavy chain variable region and the light chain variable region comprise CDRs selected from the group consisting of:
| VH-CDR1 Sequence number | VH-CDR2 Sequence number | VH-CDR3 Sequence number | VL-CDR1 Sequence number | VL-CDR2 Sequence number | VL-CDR3 Sequence number |
|---|---|---|---|---|---|
| 3 | 4 | 5 | 8 | 9 | 10 |
| 13 | 14 | 15 | 18 | 19 | 20 |
| 23 | 24 | 25 | 28 | 29 | 30 |
| 33 | 34 | 35 | 38 | 39 | 40 |
| 43 | 44 | 45 | 48 | 49 | 50 |
| 53 | 54 | 55 | 58 | 59 | 60 |
| 63 | 64 | 65 | 68 | 69 | 70 |
wherein any one of the above amino acid sequences further comprises a derivative sequence which is obtained through optional addition, deletion, modification and/or substitution of at least one amino acid and is capable of retaining the binding affinity to TIM-3.

7. The antibody of claim 5, wherein the heavy chain variable region of the antibody comprises the amino acid sequence shown in SEQ ID NO. 1, 11, 21, 31, 41, 51 or 61; and/or the light chain variable region of the antibody comprises the amino acid sequence shown in SEQ ID NO. 6, 16, 26, 36, 46, 56 or 66.

8. The antibody of claim 6, wherein the antibody is selected from the group consisting of:
| Antibody number | Clone | VH sequence number | VL sequence number |
|---|---|---|---|
| 1 | 7A4F10 | 1 | 6 |
| 2 | 18D2H2 | 11 | 16 |
| 3 | 134H3G6 | 21 | 26 |
| 4 | 215A8F2 | 31 | 36 |
| 5 | 34B6D8 | 41 | 46 |
| 6 | 39E5H1 | 51 | 56 |
| 7 | 57F4E5 | 61 | 66. |

9. A recombinant protein, wherein the recombinant protein comprises:
(i) the heavy chain variable region of claim 1, the heavy chain of claim 2, the light chain variable region of claim 3, the light chain of claim 4, or the antibody of any one of claims 5-8; and
(ii) an optional tag sequence that assists expression and/or purification.

10. A polynucleotide, wherein the polynucleotide encodes a polypeptide selected from group consisting of:
(1) the heavy chain variable region of claim 1, the heavy chain of claim 2, the light chain variable region of claim 3, the light chain of claim 4, or the antibody of any one of claims 5-8; and
(2) the recombinant protein of claim 9.

11. The polynucleotide of claim 10, wherein the polynucleotide encoding the heavy chain variable region is shown in SEQ ID NO. 2, 12, 22, 32, 42, 52 or 62; and/or, the polynucleotide encoding the light chain variable region is shown in SEQ ID NO. 7, 17, 27, 37, 47, 57 or 67.

12. The polynucleotide of claim 11, wherein the polynucleotide encoding the heavy chain variable region and the polynucleotide encoding the light chain variable region are selected from the group consisting of:
| Clone | Sequence number of the polynucleotide encoding VH | Sequence number of the polynucleotide encoding VL |
|---|---|---|
| 7A4F10 | 2 | 7 |
| 18D2H2 | 12 | 17 |
| 134H3G6 | 22 | 27 |
| 215A8F2 | 32 | 37 |
| 34B6D8 | 42 | 47 |
| 39E5H1 | 52 | 57 |
| 57F4E5 | 62 | 67. |

13. A vector, wherein the vector comprises the polynucleotide of any one of claims 10-12.

14. A genetically engineered host cell, wherein the host cell contains the vector of claim 13 or the genome thereof is integrated with the polynucleotide of any one of claims 10-12.

15. An antibody conjugate, wherein the antibody conjugate comprises:
(a) an antibody moiety, which is selected from the group consisting of: the heavy chain variable region of claim 1, the heavy chain of claim 2, the light chain variable region of claim 3, the light chain of claim 4, the antibody of any one of claims 5-8, and a combination thereof; and
(b) a coupling moiety coupled to the antibody moiety, and the coupling moiety is selected from the group consisting of a detectable label, a drug, a toxin, a cytokine, a radionuclide, an enzyme, and a combination thereof.

16. An immune cell, which expresses or is exposed outside the cell membrane with the antibody of any one of claims 5-8.

17. A pharmaceutical composition, wherein the pharmaceutical composition comprises:
(i) an active ingredient, wherein the active ingredient is selected from the group consisting of: the heavy chain variable region of claim 1, the heavy chain of claim 2, the light chain variable region of claim 3, the light chain of claim 4, and the antibody of any one of claims 5-8, the recombinant protein of claim 9, the antibody conjugate of claim 15, the immune cell of claim 16, and a combination thereof; and
(ii) a pharmaceutically acceptable carrier.


Sequence listing

<110>  SHANGHAI PHARMAEXPLORER CO., LTD.
       HARMAEXPLORER LIMITED

<120>  ANTI-HUMAN TIM-3 MONOCLONAL ANTIBODY AND APPLICATION THEREOF

<130>  P2019-2007

<150>  CN201811459248.9
<151>  2018-11-30

<160>  73

<170>  PatentIn version 3.5

<210>  1
<211>  119
<212>  PRT
<213>  Artificial sequence

<220>
<223>  Heavy chain variable region


<400>  1

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Arg Thr His
            20                  25                  30


Leu Ile Ser Trp Val Arg Gln Ala Pro Gly Lys Gly Leu Glu Trp Val
        35                  40                  45


Ala Ala Ile Thr Gly Ser Gly Gly Ser Thr Tyr Tyr Ala Asp Ser Val
    50                  55                  60


Lys Gly Arg Phe Ile Ile Ser Arg Asp Asn Ser Lys Asn Thr Leu Phe
65                  70                  75                  80


Leu Gln Met Asn Ser Leu Arg Ala Glu Asp Thr Ala Val Tyr Tyr Cys
                85                  90                  95


Ala Lys Asp Gly Gly Ser Gly Ser Tyr Asp Asp Phe Trp Gly Gln Gly
            100                 105                 110


Thr Leu Val Thr Val Ser Ser
        115


<210>  2
<211>  357
<212>  DNA
<213>  Artificial sequence

<220>
<223>  Heavy chain variable region


<400>  2
gaagtgcaac tgttggagtc tgggggaggc ttggtacagc ctggggggtc cctgagactc      60

tcctgtgcgg cctctggatt cacctttagg acccatctca taagttgggt ccgccaggct     120

ccagggaagg gactggagtg ggtcgcagct ataactggta gtggtggtag cacatactac     180

gcagactccg tgaagggccg gttcatcatc tccagagaca attccaagaa cacgctgttt     240

ctgcaaatga acagcctgag agccgaagac acggccgtat attactgtgc gaaagatggg     300

ggttcgggga gttatgatga cttctggggc cagggaaccc tggtcaccgt ctcctca        357


<210>  3
<211>  5
<212>  PRT
<213>  Artificial sequence

<220>
<223>  Heavy chain CDR1


<400>  3

Thr His Leu Ile Ser
1               5


<210>  4
<211>  17
<212>  PRT
<213>  Artificial sequence

<220>
<223>  Heavy chain CDR2


<400>  4

Ala Ile Thr Gly Ser Gly Gly Ser Thr Tyr Tyr Ala Asp Ser Val Lys
1               5                   10                  15


Gly



<210>  5
<211>  10
<212>  PRT
<213>  Artificial sequence

<220>
<223>  Heavy chain CDR3


<400>  5

Asp Gly Gly Ser Gly Ser Tyr Asp Asp Phe
1               5                   10

<210>  6
<211>  107
<212>  PRT
<213>  Artificial sequence

<220>
<223>  Light chain variable region

<400>  6

Glu Thr Val Leu Thr Gln Ser Pro Val Thr Leu Ser Leu Ser Pro Gly
1               5                   10                  15

Glu Arg Ala Thr Leu Ser Cys Arg Ala Ser Gln Ser Val Gly Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Ser Asn Trp Pro Pro
                85                  90                  95

Thr Phe Gly Gln Gly Thr Lys Val Glu Ile Lys
            100                 105

<210>  7
<211>  321
<212>  DNA
<213>  Artificial sequence

<220>
<223>  Light chain variable region

<400>  7
gaaactgtgt tgacacagtc tccagtcacc ctgtctttgt ctccagggga aagagccacc      60

ctctcctgca gggccagtca gagtgttggc agctacttag cctggtacca acagaaacct     120

ggccaggctc ccaggctcct catctatgat gcatccaaca gggccactgg catcccagcc     180

aggttcagtg gcagtgggtc tgggacagac ttcactctca ccatcagcag cctagagcct     240

gaagattttg cagtttatta ctgtcagcag cgtagcaact ggcctccgac gttcggccaa     300

gggaccaagg tggaaatcaa a                                                321

<210>  8
<211>  11
<212>  PRT
<213>  Artificial sequence

<220>
<223>  Light chain CDR1

<400>  8

Arg Ala Ser Gln Ser Val Gly Ser Tyr Leu Ala
1               5                   10

<210>  9
<211>  7
<212>  PRT
<213>  Artificial sequence

<220>
<223>  Light chain CDR2

<400>  9

Asp Ala Ser Asn Arg Ala Thr
1               5

<210>  10
<211>  9
<212>  PRT
<213>  Artificial sequence

<220>
<223>  Light chain CDR3

<400>  10

Gln Gln Arg Ser Asn Trp Pro Pro Thr
1               5

<210>  11
<211>  116
<212>  PRT
<213>  Artificial sequence

<220>
<223>  Heavy chain variable region

<400>  11

Glu Val Gln Leu Val Glu Ser Gly Gly Asp Leu Val Gln Pro Gly Gly
1               5                   10                  15

Ser Leu Arg Leu Ser Cys Ala Ala Ser Gly Phe Thr Phe Ser Thr Tyr
            20                  25                  30

Trp Met Thr Trp Val Arg Gln Ala Pro Gly Arg Gly Leu Glu Trp Val
        35                  40                  45

Ala Asn Ile Lys Gln Asp Gly Ser Ala Lys Tyr Tyr Val Asp Ser Val
    50                  55                  60

Lys Gly Arg Phe Thr Leu Ser Arg Asp Asp Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Gly Ser Asn Trp Phe Asp Tyr Trp Gly Gln Gly Thr Leu Val
            100                 105                 110

Thr Val Ser Ser
        115

<210>  12
<211>  348
<212>  DNA
<213>  Artificial sequence

<220>
<223>  Heavy chain variable region

<400>  12
gaggtgcagc tggtggagtc tgggggagac ttggtccagc ctggggggtc cctgagactc      60

tcctgtgcag cctctggatt cacctttagt acctattgga tgacgtgggt ccgccaggct     120

ccagggaggg gactggagtg ggtggccaac attaaacaag atggaagtgc gaaatactat     180

gtggactctg tgaagggccg attcaccctc tccagagacg acgccaagaa ctcactgtat     240

ctgcaaatga acagcctgag agccgaggac acggctgtgt attactgtgc gagagggagc     300

aactggtttg actactgggg ccagggaacc ctggtcaccg tctcctca                  348

<210>  13
<211>  5
<212>  PRT
<213>  Artificial sequence

<220>
<223>  Heavy chain CDR1

<400>  13

Thr Tyr Trp Met Thr
1               5

<210>  14
<211>  17
<212>  PRT
<213>  Artificial sequence

<220>
<223>  Heavy chain CDR2

<400>  14

Asn Ile Lys Gln Asp Gly Ser Ala Lys Tyr Tyr Val Asp Ser Val Lys
1               5                   10                  15

Gly

<210>  15
<211>  7
<212>  PRT
<213>  Artificial sequence

<220>
<223>  Heavy chain CDR3

<400>  15

Gly Ser Asn Trp Phe Asp Tyr
1               5

<210>  16
<211>  107
<212>  PRT
<213>  Artificial sequence

<220>
<223>  Light chain variable region

<400>  16

Glu Thr Val Met Thr Gln Ser Pro Ala Thr Leu Ser Val Ser Pro Gly
1               5                   10                  15

Glu Arg Ala Thr Leu Ser Cys Arg Ala Asn Gln Asn Val Tyr Ser Asn
            20                  25                  30

Leu Ala Trp Tyr Gln Gln Lys Pro Gly Gln Ala Pro Arg Leu Leu Ile
        35                  40                  45

Tyr Asp Ala Ser Thr Arg Ala Thr Gly Phe Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Leu
                85                  90                  95

Thr Phe Gly Gly Gly Thr Lys Val Glu Ile Lys
            100                 105

<210>  17
<211>  321
<212>  DNA
<213>  Artificial sequence

<220>
<223>  Light chain variable region

<400>  17
gaaacagtga tgacgcagtc tccagccacc ctgtctgtgt ctccagggga aagagccacc      60

ctctcctgca gggccaatca gaatgtttac agcaacttag cctggtacca gcagaaacct     120

ggccaggctc ccaggctcct catctatgat gcatccacca gggccactgg tttcccagcc     180

aggttcagtg gcagtgggtc tgggacagag ttcactctca ccatcagcag cctgcagtct     240

gaagattttg cagtttatta ctgtcagcag tataataact ggccgctcac tttcggcgga     300

gggaccaagg tggagatcaa a                                               321

<210>  18
<211>  11
<212>  PRT
<213>  Artificial sequence

<220>
<223>  Light chain CDR1

<400>  18

Arg Ala Asn Gln Asn Val Tyr Ser Asn Leu Ala
1               5                   10

<210>  19
<211>  7
<212>  PRT
<213>  Artificial sequence

<220>
<223>  Light chain CDR2

<400>  19

Asp Ala Ser Thr Arg Ala Thr
1               5

<210>  20
<211>  9
<212>  PRT
<213>  Artificial sequence

<220>
<223>  Light chain CDR3

<400>  20

Gln Gln Tyr Asn Asn Trp Pro Leu Thr
1               5

<210>  21
<211>  116
<212>  PRT
<213>  Artificial sequence

<220>
<223>  Heavy chain variable region

<400>  21

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asn Ser
            20                  25                  30

Trp Met Thr Trp Val Arg Gln Ala Pro Gly Lys Gly Leu Glu Trp Val
        35                  40                  45

Ala Asn Ile Lys Gln Ala Gly Thr Glu Lys Tyr Tyr Val Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Asn Ser Gly Ser Asn Tyr Phe Asp Tyr Trp Gly Gln Gly Thr Leu Val
            100                 105                 110

Thr Val Ser Ser
        115

<210>  22

<211>  348
<212>  DNA
<213>  Artificial sequence

<220>
<223>  Heavy chain variable region


<400>  22
gaggtgcagc tggtggagtc tgggggaggc ttggtccagc ctggggggtc cctgagactc      60

tcctgtgcag cctctggatt cacctttagt aactcttgga tgacctgggt ccgccaggct     120

ccagggaagg ggctggagtg ggtggccaac ataaagcaag ctggaactga gaaatactat     180

gtggactctg tgaagggccg attcaccatc tccagagaca acgccaagaa ctcactgtat     240

ctgcaaatga acagcctgag agccgaggac acggctgtgt attactgtaa tagtgggagc     300

aactactttg actactgggg ccagggaacc ctggtcaccg tctcctca                  348


<210>  23
<211>  5
<212>  PRT
<213>  Artificial sequence

<220>
<223>  Heavy chain CDR1


<400>  23

Asn Ser Trp Met Thr
1               5


<210>  24
<211>  17
<212>  PRT
<213>  Artificial sequence

<220>
<223>  Heavy chain CDR2


<400>  24

Asn Ile Lys Gln Ala Gly Thr Glu Lys Tyr Tyr Val Asp Ser Val Lys
1               5                   10                  15


Gly



<210>  25
<211>  7
<212>  PRT
<213>  Artificial sequence

<220>
<223>  Heavy chain CDR3

<400>  25

Gly Ser Asn Tyr Phe Asp Tyr
1               5

<210>  26
<211>  107
<212>  PRT
<213>  Artificial sequence

<220>
<223>  Light chain variable region

<400>  26

Glu Ile Val Met Thr Gln Ser Pro Ala Thr Leu Ser Val Ser Pro Gly
1               5                   10                  15

Glu Arg Ala Thr Leu Ser Cys Arg Ala Arg Gln Asn Phe Tyr Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Leu
                85                  90                  95

Thr Phe Gly Gly Gly Thr Lys Val Glu Ile Lys
            100                 105

<210>  27
<211>  321
<212>  DNA
<213>  Artificial sequence

<220>
<223>  Light chain variable region

<400>  27
gaaatagtaa tgacgcagtc tccagccacc ctgtctgtgt ctccagggga aagagccacc      60

ctctcctgca gggccagaca gaatttttac agcaacttag cctggtacca acagaaacct     120

ggccaggctc ccaggctcct catctatggt gcatccacca gggccactgg tatcccagcc     180

aggttcagtg gcagtgggtc tgggacagag ttcactctca ccatcagcag cctgcagtct     240

gaagattttg cagtttatta ctgtcagcag tataataact ggcctctcac tttcggcgga     300

gggaccaagg tggagatcaa a                                               321


<210>  28
<211>  11
<212>  PRT
<213>  Artificial sequence

<220>
<223>  Light chain CDR1


<400>  28

Arg Ala Arg Gln Asn Phe Tyr Ser Asn Leu Ala
1               5                   10


<210>  29
<211>  7
<212>  PRT
<213>  Artificial sequence

<220>
<223>  Light chain CDR2


<400>  29

Gly Ala Ser Thr Arg Ala Thr
1               5


<210>  30
<211>  9
<212>  PRT
<213>  Artificial sequence

<220>
<223>  Light chain CDR3


<400>  30

Gln Gln Tyr Asn Asn Trp Pro Leu Thr
1               5


<210>  31
<211>  117
<212>  PRT
<213>  Artificial sequence

<220>
<223>  Heavy chain variable region


<400>  31

Gln Val Gln Leu Gln Gln Trp Gly Ala Gly Leu Leu Lys Pro Ser Glu
1               5                   10                  15

Thr Leu Ser Leu Thr Cys Thr Val Tyr Gly Gly Ser Phe Ser Gly Tyr
            20                  25                  30

Tyr Trp Asn Trp Ile Arg Gln Pro Pro Gly Lys Gly Leu Glu Trp Leu
        35                  40                  45

Gly Glu Ile Asn His Ser Gly Ser Thr Asn Tyr Asn Pro Ser Leu Lys
    50                  55                  60

Arg Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Val Tyr Tyr Tyr Gly Pro Phe Asp Phe Trp Gly Gln Gly Thr Leu
            100                 105                 110

Val Thr Val Ser Ser
        115

<210>  32
<211>  351
<212>  DNA
<213>  Artificial sequence

<220>
<223>  Heavy chain variable region

<400>  32
caggtgcagc tacagcagtg gggcgcagga ctgttgaagc cttcggagac cctgtccctc      60

acctgcactg tctatggtgg gtccttcagt ggttactact ggaactggat ccgccagccc     120

ccagggaagg ggctggagtg gcttggggaa atcaatcata gtggaagcac caactacaac     180

ccgtccctca agagacgagt caccatatca gtggacacgt ccaagaacca gttctccctg     240

aagctgagct ctgtgaccgc cgcggacacg gctgtgtatt actgtgcgag ggtgtattat     300

tatggccctt ttgacttctg gggccaggga accctggtca ccgtctcctc a              351

<210>  33
<211>  5
<212>  PRT
<213>  Artificial sequence

<220>
<223>  Heavy chain CDR1

<400>  33

Gly Tyr Tyr Trp Asn
1               5


<210>  34
<211>  16
<212>  PRT
<213>  Artificial sequence

<220>
<223>  Heavy chain CDR2


<400>  34

Glu Ile Asn His Ser Gly Ser Thr Asn Tyr Asn Pro Ser Leu Lys Arg
1               5                   10                  15


<210>  35
<211>  9
<212>  PRT
<213>  Artificial sequence

<220>
<223>  Heavy chain CDR3


<400>  35

Val Tyr Tyr Tyr Gly Pro Phe Asp Phe
1               5


<210>  36
<211>  113
<212>  PRT
<213>  Artificial sequence

<220>
<223>  Light chain variable region


<400>  36

Asp Ile Val Met Thr Gln Ser Pro Asp Ser Leu Ala Val Ser Leu Gly
1               5                   10                  15


Glu Arg Ala Thr Ile Asn Cys Lys Ser Ser Gln Ser Val Leu Tyr Arg
            20                  25                  30


Ser Asn Asn Lys Asn Tyr Leu Ala Trp Tyr Gln Gln Lys Pro Gly Gln
        35                  40                  45


Pro Pro Lys Leu Leu Ile Tyr Trp Ala Ser Thr Arg Glu Ser Gly Val
    50                  55                  60

Pro Asp Arg Phe Ser Gly Ser Gly Pro Gly Thr Asp Phe Thr Leu Thr
65                  70                  75                  80

Ile Asn Ser Leu Gln Ala Glu Asp Val Ala Val Tyr Tyr Cys Gln Gln
                85                  90                  95

Tyr Tyr Ser Thr Pro Tyr Thr Phe Gly Gln Gly Thr Lys Leu Glu Ile
            100                 105                 110

Lys

<210>  37
<211>  339
<212>  DNA
<213>  Artificial sequence

<220>
<223>  Light chain variable region

<400>  37
gacatcgtga tgacccagtc tccagactcc ctggctgtgt ctctgggcga gagggccacc      60

atcaactgca agtccagcca gagtgtttta tacaggtcca acaataagaa ctacttagct     120

tggtaccagc agaaaccagg acagcctcct aagctgctca tttactgggc atctacccgg     180

gaatccgggg tccctgaccg attcagtggc agcgggcctg ggacagattt cactctcacc     240

atcaacagcc tgcaggctga agatgtggca gtttattact gtcagcaata ttatagtact     300

ccgtacactt ttggccaggg gaccaagctg gagatcaaa                            339

<210>  38
<211>  17
<212>  PRT
<213>  Artificial sequence

<220>
<223>  Light chain CDR1

<400>  38

Lys Ser Ser Gln Ser Val Leu Tyr Arg Ser Asn Asn Lys Asn Tyr Leu
1               5                   10                  15

Ala

<210>  39
<211>  7
<212>  PRT
<213>  Artificial sequence

<220>
<223>  Light chain CDR2


<400>  39

Trp Ala Ser Thr Arg Glu Ser
1               5


<210>  40
<211>  9
<212>  PRT
<213>  Artificial sequence

<220>
<223>  Light chain CDR3


<400>  40

Gln Gln Tyr Tyr Ser Thr Pro Tyr Thr
1               5


<210>  41
<211>  116
<212>  PRT
<213>  Artificial sequence

<220>
<223>  Heavy chain variable region


<400>  41

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Thr Tyr
            20                  25                  30


Trp Met Thr Trp Val Arg Gln Ala Pro Gly Lys Arg Leu Glu Trp Val
        35                  40                  45


Ala Asn Ile Lys Gln Gly Gly Gly Asp Lys Tyr Tyr Val Asp Ser Val
    50                  55                  60


Lys Gly Arg Phe Thr Ile Ser Thr Asp Asn Ala Lys Asn Ser Leu Tyr
65                  70                  75                  80


Leu Gln Met Asn Ser Leu Arg Ala Glu Asp Thr Ala Val Tyr Tyr Cys
                85                  90                  95


Ala Arg Gly Ser Asn Trp Phe Asp Pro Trp Gly Gln Gly Thr Leu Val
            100                 105                 110

Thr Val Ser Ser
        115

<210>  42
<211>  348
<212>  DNA
<213>  Artificial sequence

<220>
<223>  Heavy chain variable region

<400>  42
gaggtgcagc tggtggagtc tgggggaggc ttggtccagc ctggggggtc cctgcgtctc      60

tcctgtgcag cctctggatt cacctttagt acctattgga tgacctgggt ccgccaggct     120

ccagggaagc ggctggagtg ggtggccaac ataaagcaag gtggaggtga taaatactat     180

gtggactctg tgaagggccg attcaccatc tccacagaca acgccaagaa ctcactgtat     240

ctgcaaatga acagcctgag agccgaggac acggctgtgt attactgtgc gagagggtcc     300

aactggttcg acccctgggg ccagggaacc ctggtcaccg tctcctca                  348

<210>  43
<211>  5
<212>  PRT
<213>  Artificial sequence

<220>
<223>  Heavy chain CDR1

<400>  43

Thr Tyr Trp Met Thr
1               5

<210>  44
<211>  17
<212>  PRT
<213>  Artificial sequence

<220>
<223>  Heavy chain CDR2

<400>  44

Asn Ile Lys Gln Gly Gly Gly Asp Lys Tyr Tyr Val Asp Ser Val Lys
1               5                   10                  15

Gly

<210>  45
<211>  7
<212>  PRT
<213>  Artificial sequence

<220>
<223>  Heavy chain CDR3


<400>  45

Gly Ser Asn Trp Phe Asp Pro
1               5


<210>  46
<211>  107
<212>  PRT
<213>  Artificial sequence

<220>
<223>  Light chain variable region


<400>  46

Glu Ile Val Met Thr Gln Ser Pro Ala Thr Leu Ser Met Ser Pro Gly
1               5                   10                  15


Glu Arg Ala Thr Leu Ser Cys Arg Ala Ser Gln Ser Val Tyr Asp Asn
            20                  25                  30


Leu Ala Trp Tyr Gln Gln Lys Pro Gly Gln Ala Pro Arg Leu Leu Ile
        35                  40                  45


Tyr Gly Ala Ser Thr Arg Ala Thr Gly Ile Pro Ala Arg Phe Ser Gly
    50                  55                  60


Ser Gly Ser Gly Thr Glu Phe Thr Leu Thr Ile Ser Ser Leu Gln Ser
65                  70                  75                  80


Glu Asp Phe Ala Leu Tyr Tyr Cys Gln Gln Tyr Thr His Trp Pro Ile
                85                  90                  95


Thr Phe Gly Gln Gly Thr Arg Leu Glu Ile Lys
            100                 105


<210>  47
<211>  321
<212>  DNA
<213>  Artificial sequence

<220>
<223>  Light chain variable region

<400>  47
gaaatagtga tgacgcagtc tccagccacc ctgtctatgt ctccagggga aagagccacc      60

ctctcctgca gggccagtca gagtgtttac gacaacttag cctggtacca gcagaaacct     120

ggccaggctc ccaggctcct catctatggt gcatccacca gggccactgg tatcccagcc     180

aggttcagtg gcagtgggtc tgggacagag ttcactctca ccatcagcag cctgcagtct     240

gaagattttg cactttatta ctgtcagcag tatactcact ggccgatcac cttcggccaa     300

gggacacgac tggagattaa a                                               321


<210>  48
<211>  11
<212>  PRT
<213>  Artificial sequence

<220>
<223>  Light chain CDR1


<400>  48

Arg Ala Ser Gln Ser Val Tyr Asp Asn Leu Ala
1               5                   10


<210>  49
<211>  7
<212>  PRT
<213>  Artificial sequence

<220>
<223>  Light chain CDR2


<400>  49

Gly Ala Ser Thr Arg Ala Thr
1               5


<210>  50
<211>  9
<212>  PRT
<213>  Artificial sequence

<220>
<223>  Light chain CDR3


<400>  50

Gln Gln Tyr Thr His Trp Pro Ile Thr
1               5


<210>  51
<211>  116
<212>  PRT
<213>  Artificial sequence

<220>
<223>  Heavy chain variable region

<400>  51

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ala Asn Ile Lys Arg Gly Gly Ser Glu Arg Tyr Tyr Val Asp Ser Val
    50                  55                  60

Lys Gly Arg Phe Thr Ile Ser Gly Asp Asn Ala Lys Asn Ser Val Tyr
65                  70                  75                  80

Leu Gln Met Asn Ser Leu Arg Ala Glu Asp Thr Ala Val Tyr Tyr Cys
                85                  90                  95

Ala Arg Gly Ser Asn Trp Phe Asp Pro Trp Gly Gln Gly Thr Leu Val
            100                 105                 110

Thr Val Ser Ser
        115

<210>  52
<211>  348
<212>  DNA
<213>  Artificial sequence

<220>
<223>  Heavy chain variable region

<400>  52
gaggtgcagc tggtggagtc tgggggaggc ttggtccagc ctggggggtc cctgagactc      60

tcctgtgcag cctctggatt cacctttagt agctattgga tgagctgggt ccgccaggct     120

ccagggaagg gactggagtg ggtggccaac ataaagcgag gtggaagtga gagatactat     180

gtggactctg tgaagggccg attcaccatc tccggagaca acgccaagaa ctcagtgtat     240

ctgcaaatga acagcctgag agccgaggac acggctgtgt attactgtgc gagaggctcc     300

aactggttcg acccctgggg ccagggaacc ctggtcaccg tctcctca                  348

<210>  53

<211>  5
<212>  PRT
<213>  Artificial sequence

<220>
<223>  Heavy chain CDR1


<400>  53

Ser Tyr Trp Met Ser
1               5


<210>  54
<211>  17
<212>  PRT
<213>  Artificial sequence

<220>
<223>  Heavy chain CDR2


<400>  54

Asn Ile Lys Arg Gly Gly Ser Glu Arg Tyr Tyr Val Asp Ser Val Lys
1               5                   10                  15


Gly




<210>  55
<211>  7
<212>  PRT
<213>  Artificial sequence

<220>
<223>  Heavy chain CDR3


<400>  55

Gly Ser Asn Trp Phe Asp Pro
1               5


<210>  56
<211>  107
<212>  PRT
<213>  Artificial sequence

<220>
<223>  Light chain variable region


<400>  56

Glu Thr Val Met Thr Gln Ser Pro Ala Ile Leu Ser Val Ser Pro Gly
1               5                   10                  15

Glu Arg Ala Thr Leu Ser Cys Arg Ala Ser Gln Ser Val Tyr Ser Asn
            20                  25                  30

Leu Ala Trp Tyr Gln Gln Lys Leu Gly Gln Ala Pro Arg Leu Leu Ile
        35                  40                  45

Tyr Asp Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Ile
                85                  90                  95

Thr Phe Gly Gln Gly Thr Arg Leu Glu Ile Lys
            100                 105

<210>  57
<211>  321
<212>  DNA
<213>  Artificial sequence

<220>
<223>  Light chain variable region

<400>  57
gaaacagtga tgacgcagtc tccagccatc ctgtctgtgt ctccagggga aagagccacc      60

ctctcctgca gggccagtca gagtgtttac agcaacttag cctggtacca acagaaactt     120

ggccaggctc ccaggctcct catctatgat gcatccacca gggccactgg tatcccagcc     180

aggttcagtg gcagtgggtc tgggacagag ttcactctca ccatcagcag cctgcagtct     240

gaagattttg cagtttatta ctgtcagcag tataataact ggccgatcac cttcggccaa     300

gggacacgac tggagattaa a                                               321

<210>  58
<211>  11
<212>  PRT
<213>  Artificial sequence

<220>
<223>  Light chain CDR1

<400>  58

Arg Ala Ser Gln Ser Val Tyr Ser Asn Leu Ala
1               5                   10

<210>  59

<211>  7
<212>  PRT
<213>  Artificial sequence

<220>
<223>  Light chain CDR2


<400>  59

Asp Ala Ser Thr Arg Ala Thr
1               5


<210>  60
<211>  9
<212>  PRT
<213>  Artificial sequence

<220>
<223>  Light chain CDR3


<400>  60

Gln Gln Tyr Asn Asn Trp Pro Ile Thr
1               5


<210>  61
<211>  116
<212>  PRT
<213>  Artificial sequence

<220>
<223>  Heavy chain variable region


<400>  61

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Trp Met Ser Trp Val Arg Gln Ala Pro Gly Lys Gly Leu Glu Trp Val
        35                  40                  45


Ala Asn Ile Lys Gln Asp Gly Ser Glu Lys Tyr Tyr Val Asp Ser Val
    50                  55                  60


Lys Gly Arg Phe Thr Ile Ser Arg Asp Ser Ala Lys Asn Ser Leu Tyr
65                  70                  75                  80


Leu Gln Met Asn Ser Leu Arg Ala Glu Asp Thr Ala Met Tyr Phe Cys
                85                  90                  95

Ala Arg Asp Ser Asn Phe Phe Asp Tyr Trp Gly Gln Gly Thr Leu Val
            100                 105                 110

Thr Val Ser Ser
        115

<210>  62
<211>  348
<212>  DNA
<213>  Artificial sequence

<220>
<223>  Heavy chain variable region

<400>  62
gaggtgcagc tggtggagtc tgggggaggc ttggtccagc ctggggggtc cctgagactc        60

tcctgtgcag cctctggatt cacctttagt agctattgga tgagctgggt ccgccaggct       120

ccagggaagg ggctggagtg ggtggccaac ataaagcaag atgggagtga gaaatactat       180

gtggactctg tgaagggccg attcaccatc tccagagaca gcgccaagaa ctcactgtat       240

cttcaaatga acagcctgag agccgaggac acggctatgt atttctgtgc gagagactcc       300

aacttctttg actactgggg ccagggaacc ctggtcaccg tctcctca                    348

<210>  63
<211>  5
<212>  PRT
<213>  Artificial sequence

<220>
<223>  Heavy chain CDR1

<400>  63

Ser Tyr Trp Met Ser
1               5

<210>  64
<211>  17
<212>  PRT
<213>  Artificial sequence

<220>
<223>  Heavy chain CDR2

<400>  64

Asn Ile Lys Gln Asp Gly Ser Glu Lys Tyr Tyr Val Asp Ser Val Lys
1               5                   10                  15

Gly

<210>  65
<211>  7
<212>  PRT
<213>  Artificial sequence

<220>
<223>  Heavy chain CDR3


<400>  65

Asp Ser Asn Phe Phe Asp Tyr
1               5


<210>  66
<211>  107
<212>  PRT
<213>  Artificial sequence

<220>
<223>  Light chain variable region


<400>  66

Glu Thr Val Met Thr Gln Ser Pro Ala Thr Leu Ser Val Ser Pro Gly
1               5                   10                  15


Glu Arg Ala Thr Leu Ser Cys Arg Ala Ser Gln Ser Val Ser Ser Asn
            20                  25                  30


Leu Ala Trp Tyr Gln Arg Lys Pro Gly Gln Ala Pro Arg Leu Leu Ile
        35                  40                  45


Tyr Phe Ser Ser Thr Arg Ala Thr Gly Ile Pro Ala Arg Phe Ser Gly
    50                  55                  60


Ser Gly Ser Gly Thr Glu Phe Thr Leu Thr Ile Ser Ser Leu Gln Ser
65                  70                  75                  80


Glu Asp Phe Ala Val Tyr Tyr Cys Gln Gln Tyr Asn Asn Trp Pro Leu
                85                  90                  95


Thr Phe Gly Gly Gly Thr Lys Val Glu Ile Lys
            100                 105


<210>  67
<211>  321
<212>  DNA
<213>  Artificial sequence

<220>

<223>  Light chain variable region

<400>  67
gaaacagtga tgacgcagtc tccagccacc ctgtctgtgt ctccagggga aagagccacc      60

ctctcctgca gggccagtca gagtgttagc agcaacttag cctggtacca gcggaaacct     120

ggccaggctc ccaggctcct catctatttt tcatccacca gggccactgg aatcccagcc     180

aggttcagtg gcagtgggtc tgggacagag ttcactctca ccatcagcag cctgcagtct     240

gaagattttg cagtttatta ctgtcagcag tataataact ggccgctcac tttcggcgga     300

gggaccaagg tggagatcaa a                                               321

<210>  68
<211>  11
<212>  PRT
<213>  Artificial sequence

<220>
<223>  Light chain CDR1

<400>  68

Arg Ala Ser Gln Ser Val Ser Ser Asn Leu Ala
1               5                   10

<210>  69
<211>  7
<212>  PRT
<213>  Artificial sequence

<220>
<223>  Light chain CDR2

<400>  69

Phe Ser Ser Thr Arg Ala Thr
1               5

<210>  70
<211>  9
<212>  PRT
<213>  Artificial sequence

<220>
<223>  Light chain CDR3

<400>  70

Gln Gln Tyr Asn Asn Trp Pro Leu Thr
1               5

<210>  71

<211>  301
<212>  PRT
<213>  Artificial sequence

<220>
<223>  Human TIM-3


<400>  71

Met Phe Ser His Leu Pro Phe Asp Cys Val Leu Leu Leu Leu Leu Leu
1               5                   10                  15

Leu Leu Thr Arg Ser Ser Glu Val Glu Tyr Arg Ala Glu Val Gly Gln
            20                  25                  30

Asn Ala Tyr Leu Pro Cys Phe Tyr Thr Pro Ala Ala Pro Gly Asn Leu
        35                  40                  45

Val Pro Val Cys Trp Gly Lys Gly Ala Cys Pro Val Phe Glu Cys Gly
    50                  55                  60

Asn Val Val Leu Arg Thr Asp Glu Arg Asp Val Asn Tyr Trp Thr Ser
65                  70                  75                  80

Arg Tyr Trp Leu Asn Gly Asp Phe Arg Lys Gly Asp Val Ser Leu Thr
                85                  90                  95

Ile Glu Asn Val Thr Leu Ala Asp Ser Gly Ile Tyr Cys Cys Arg Ile
            100                 105                 110

Gln Ile Pro Gly Ile Met Asn Asp Glu Lys Phe Asn Leu Lys Leu Val
        115                 120                 125

Ile Lys Pro Ala Lys Val Thr Pro Ala Pro Thr Arg Gln Arg Asp Phe
    130                 135                 140

Thr Ala Ala Phe Pro Arg Met Leu Thr Thr Arg Gly His Gly Pro Ala
145                 150                 155                 160

Glu Thr Gln Thr Leu Gly Ser Leu Pro Asp Ile Asn Leu Thr Gln Ile
                165                 170                 175

Ser Thr Leu Ala Asn Glu Leu Arg Asp Ser Arg Leu Ala Asn Asp Leu
            180                 185                 190

Arg Asp Ser Gly Ala Thr Ile Arg Ile Gly Ile Tyr Ile Gly Ala Gly
        195                 200                 205

Ile Cys Ala Gly Leu Ala Leu Ala Leu Ile Phe Gly Ala Leu Ile Phe

210                 215                 220

Lys Trp Tyr Ser His Ser Lys Glu Lys Ile Gln Asn Leu Ser Leu Ile
225                 230                 235                 240

Ser Leu Ala Asn Leu Pro Pro Ser Gly Leu Ala Asn Ala Val Ala Glu
                245                 250                 255

Gly Ile Arg Ser Glu Glu Asn Ile Tyr Thr Ile Glu Glu Asn Val Tyr
            260                 265                 270

Glu Val Glu Glu Pro Asn Glu Tyr Tyr Cys Tyr Val Ser Ser Arg Gln
        275                 280                 285

Gln Pro Ser Gln Pro Leu Gly Cys Arg Phe Ala Met Pro
    290                 295                 300

<210>  72
<211>  302
<212>  PRT
<213>  Artificial sequence

<220>
<223>  Monkey TIM-3

<400>  72

Met Phe Ser His Leu Pro Phe Asp Cys Val Leu Leu Leu Leu Leu Leu
1               5                   10                  15

Leu Leu Thr Arg Ser Ser Glu Val Glu Tyr Ile Ala Glu Val Gly Gln
            20                  25                  30

Asn Ala Tyr Leu Pro Cys Ser Tyr Thr Pro Ala Pro Pro Gly Asn Leu
        35                  40                  45

Val Pro Val Cys Trp Gly Lys Gly Ala Cys Pro Val Phe Asp Cys Ser
    50                  55                  60

Asn Val Val Leu Arg Thr Asp Asn Arg Asp Val Asn Asp Arg Thr Ser
65                  70                  75                  80

Gly Arg Tyr Trp Leu Lys Gly Asp Phe His Lys Gly Asp Val Ser Leu
                85                  90                  95

Thr Ile Glu Asn Val Thr Leu Ala Asp Ser Gly Val Tyr Cys Cys Arg
            100                 105                 110

Ile Gln Ile Pro Gly Ile Met Asn Asp Glu Lys His Asn Val Lys Leu

115                 120                 125

Val Val Ile Lys Pro Ala Lys Val Thr Pro Ala Pro Thr Leu Gln Arg
    130                 135                 140

Asp Leu Thr Ser Ala Phe Pro Arg Met Leu Thr Thr Gly Glu His Gly
145                 150                 155                 160

Pro Ala Glu Thr Gln Thr Pro Gly Ser Leu Pro Asp Val Asn Leu Thr
                165                 170                 175

Val Ser Asn Phe Phe Cys Glu Leu Gln Ile Phe Thr Leu Thr Asn Glu
            180                 185                 190

Leu Arg Asp Ser Gly Ala Thr Ile Arg Thr Ala Ile Tyr Ile Ala Ala
        195                 200                 205

Gly Ile Ser Ala Gly Leu Ala Leu Ala Leu Ile Phe Gly Ala Leu Ile
    210                 215                 220

Phe Lys Trp Tyr Ser His Ser Lys Glu Lys Thr Gln Asn Leu Ser Leu
225                 230                 235                 240

Ile Ser Leu Ala Asn Ile Pro Pro Ser Gly Leu Ala Asn Ala Val Ala
                245                 250                 255

Glu Gly Ile Arg Ser Glu Glu Asn Ile Tyr Thr Ile Glu Glu Asp Val
            260                 265                 270

Tyr Glu Val Glu Glu Pro Asn Glu Tyr Tyr Cys Tyr Val Ser Ser Gly
        275                 280                 285

Gln Gln Pro Ser Gln Pro Leu Gly Cys Arg Val Ala Met Pro
    290                 295                 300

<210>  73
<211>  281
<212>  PRT
<213>  Artificial sequence

<220>
<223>  Mouse TIM-3

<400>  73

Met Phe Ser Gly Leu Thr Leu Asn Cys Val Leu Leu Leu Leu Gln Leu
1               5                   10                  15

Leu Leu Ala Arg Ser Leu Glu Asp Gly Tyr Lys Val Glu Val Gly Lys

20                  25                  30

Asn Ala Tyr Leu Pro Cys Ser Tyr Thr Leu Pro Thr Ser Gly Thr Leu
        35                  40                  45

Val Pro Met Cys Trp Gly Lys Gly Phe Cys Pro Trp Ser Gln Cys Thr
    50                  55                  60

Asn Glu Leu Leu Arg Thr Asp Glu Arg Asn Val Thr Tyr Gln Lys Ser
65                  70                  75                  80

Ser Arg Tyr Gln Leu Lys Gly Asp Leu Asn Lys Gly Asp Val Ser Leu
                85                  90                  95

Ile Ile Lys Asn Val Thr Leu Asp Asp His Gly Thr Tyr Cys Cys Arg
            100                 105                 110

Ile Gln Phe Pro Gly Leu Met Asn Asp Lys Lys Leu Glu Leu Lys Leu
        115                 120                 125

Asp Ile Lys Ala Ala Lys Val Thr Pro Ala Gln Thr Ala His Gly Asp
    130                 135                 140

Ser Thr Thr Ala Ser Pro Arg Thr Leu Thr Thr Glu Arg Asn Gly Ser
145                 150                 155                 160

Glu Thr Gln Thr Leu Val Thr Leu His Asn Asn Asn Gly Thr Lys Ile
                165                 170                 175

Ser Thr Trp Ala Asp Glu Ile Lys Asp Ser Gly Glu Thr Ile Arg Thr
            180                 185                 190

Ala Ile His Ile Gly Val Gly Val Ser Ala Gly Leu Thr Leu Ala Leu
        195                 200                 205

Ile Ile Gly Val Leu Ile Leu Lys Trp Tyr Ser Cys Lys Lys Lys Lys
    210                 215                 220

Leu Ser Ser Leu Ser Leu Ile Thr Leu Ala Asn Leu Pro Pro Gly Gly
225                 230                 235                 240

Leu Ala Asn Ala Gly Ala Val Arg Ile Arg Ser Glu Glu Asn Ile Tyr
                245                 250                 255

Thr Ile Glu Glu Asn Val Tyr Glu Val Glu Asn Ser Asn Glu Tyr Tyr
            260                 265                 270

Cys Tyr Val Asn Ser Gln Gln Pro Ser
        275                 280